# EUROPEAN PATENT SPECIFICATION

(11) **EP 4 267 540 B1**
(45) Date of publication and mention of the grant of the patent: **24.06.2026**
(21) Application number: 22703982.3
(22) Date of filing: 02.02.2022
(51) Int. Cl.: C07C 6/02, C07F 15/00, B01J 31/22

(54) **LONG SHELF LIFE STABLE ORGANORUTHENIUM COMPLEXES AS (PRE)CATALYSTS FOR OLEFIN METATHESIS**
LAGERSTABILE ORGANORUTHENIUMKOMPLEXE ALS (PRÄ)KATALYSATOREN FÜR DIE OLEFINMETATHESE
COMPLEXES D'ORGANORUTHÉNIUM STABLES À LONGUE DURÉE DE CONSERVATION EN TANT QUE (PRÉ)CATALYSEURS POUR LA MÉTATHÈSE D'OLÉFINES

(30) Priority: 02.02.2021 EP 21154875; 02.02.2021 US 202163144610 P
(43) Date of publication of application: 01.11.2023
(73) Proprietor: Apeiron Synthesis S.A., 54-427 Wroclaw (PL)
(72) Inventor: CHWALBA, Michal, 53-030 Wroclaw (PL); SKOWERSKI, Krzysztof, 87-330 Jablonowo Pomorskie (PL); KURCBACH, Konrad, 62-874 Brzeziny (PL)
(74) Representative: AOMB Polska Sp. z.o.o.
(86) International application number: PCT/IB2022/050893
(87) International publication number: WO 2022/167944

(56) References cited:
- ÖZTÜRK BENGI ÖZGÜN ET AL: "In situ modification of the Grubbs first generation catalyst: A highly controllable metathesis catalyst bearing tridentate Schiff base ligands", JOURNAL OF MOLECULAR CATALYSIS A: CHEMICAL, ELSEVIER, AMSTERDAM, NL, vol. 376, 17 April 2013 (2013-04-17), pages 53 - 62, XP028565217, ISSN: 1381-1169, DOI: 10.1016/J.MOLCATA.2013.04.010

## Description

### Field of the Invention

The present invention relates to a series of long shelf life stable metal complexes, their use as (pre)catalysts in the metathesis reaction as well as the process for carrying out the metathesis reaction. More specifically, the present invention relates to a series of organoruthenium compounds which exhibit long shelf life stability and when activated under suitable conditions exhibit high catalytic activity for a wide range of metathesis reactions. This invention also relates to methods of making these compounds. Accordingly, the compounds of this invention find utility as (pre)catalysts for carrying out a variety of olefin metathesis reactions, including ring-opening metathetic polymerization (ROMP), among others.

### Background art

The metathesis of olefins is an important tool in the organic synthesis (R. H. Grubbs (Ed.), AG Wenzel (Ed.), D. J. O'Leary (Ed.), E. Khosravi (Ed.), Handbook of Olefin Metathesis, 2nd edition, 3 Volumes, 2015, John Wiley & Sons, Inc. 1608 pages]).

Many ruthenium complexes actively catalyzing the olefin metathesis reactions are well known in the art (see, for example, Vougioukalakis, G. C.; Grubbs, R. H. Chem. Rev. 2010, 110, 1746). The third generation complexes (such as Gru-III, Ind-III) were shown to be highly useful (pre)catalysts of the ring-opening metathetic polymerization (ROMP) reaction.

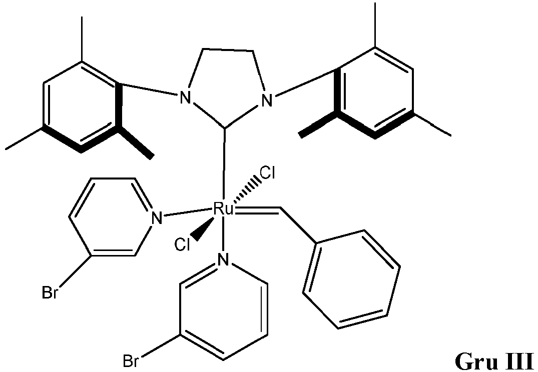

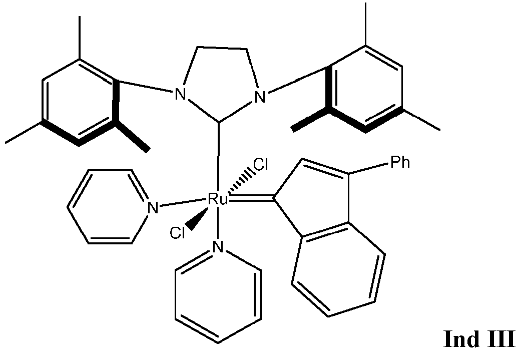

The third-generation catalysts initiate the metathesis reactions very promptly, whereas, in some metathesis applications, such as mold ROMP polymerization, it is advantageous to use a (pre)catalyst that does not initiate the reaction immediately after adding it to the substrate but only after an appropriate initiation by chemical agents, temperature or light. The complexes characterized by delayed initiation are often termed "dormant catalysts" or "latent catalysts" (Monsaert, S.; Vila, A. L.; Drozdzak, R.; Van Der Voort, P.; Verpoort, F., Chem. Soc. Rev., 2009, 38, 3360; R. Drozdzak, N. Nishioka, G. Recher, F. Verpoort, Macromol. Symp. 2010, 293, 1-4). Exemplary "dormant catalysts" are the complexes A-F, as well as the recently obtained P-1 and P-2 (Pietraszuk, C.; Rogalski, S.; Powala, B.; Mitkiewski, M.; Kubicki, M.; Spolnik, G.; Danikiewicz, W.; Wozniak, K.; Pazio, A.; Szadkowska, A.; Kozlowska, A.; Grela, K., Chem. Eur. J, 2012, 18, 6465-6469).

The mold ROMP polymerization allows obtaining finished articles. Dicyclopentadiene is one of the monomers frequently used for the mold polymerization. Polydicyclopentadiene, being obtained by polymerization of dicyclopentadiene, features, inter alia, a low moisture absorption as well as resistance to stress and high temperature. This is why parts of vehicles and specialized containers for the chemical industry are more and more frequently manufactured by the (mold) ROMP polymerization of dicyclopentadiene.

U. S. Patent No. 9,328,132 B2 addresses some of these deficiencies faced by the art in providing more robust "dormant catalysts" for olefin metathesis reactions. However, there is still a need for improved "dormant catalysts" which can be activated under desirable ROMP polymerization conditions and based on the intended end applications.

Öztürk Bengi, Özgün et al in J. Mol. Cat., vol. 376, 2013, p. 53-62 disclose olefin metathesis catalysts in form of ruthenium complexes that are modified Grubbs-type catalysts with tridentate Schiff base (-O-N-O-) bis anionic ligand. These are extremely latent and thus require thermal and chemical activation at the same time. The chemical activation was achieved using HCl. The paper discloses use of these complexes as catalysts in ROMP of cyclooctene (COE) and RCM (ring closing metathesis).

Moreover, there is still a need for latent catalysts which are dormant and can readily be activated with higher activity upon activation.

Accordingly, it is an object of this invention to provide a series of improved "dormant catalysts," which are stable at ambient temperatures for several months on the shell (as solid) as well as in the mixture with highly reactive monomer(s) (e.g. DCPD), and exhibit higher activity only upon activation.

It is also an object of this invention to provide processes for the preparation of such organoruthenium dormant catalysts as disclosed herein.

Other objects and further scope of the applicability of the present invention will become apparent from the detailed description that follows.

From the viewpoint of practical industrial applications, it is of extreme importance that the (pre)catalysts are stable in the presence of oxygen as well as moisture, during both their synthesis, purification, preparation of mixture with monomer, handling, storing, transporting it and also during their use in the metathesis reaction. Development of stable and active (pre)catalysts for metathesis of olefins as reported in the literature allowed to broaden significantly the scope of possible uses of this transformation. Nevertheless, these complexes are still prepared and used in metathesis reactions in atmosphere of inert gas, in dry solvents, since their stability against oxygen and moisture is limited.

Invention relates to the organoruthenium compound of the formula (II): 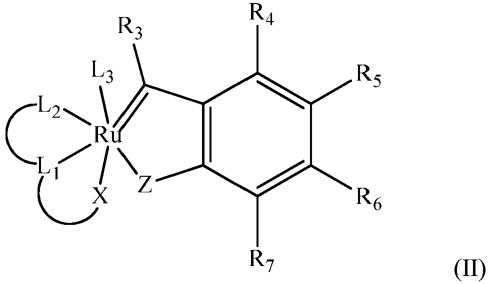 wherein ,.
the present invention relates to the compounds of formula (II), including all possible enantiomers, diastereoisomers, geometric or any other stereoisomers thereof, wherein:
Z is oxygen;
R₃ is hydrogen;
R₄, R₅, R₆ and R₇ are the same or different and each independently selected from the group consisting of hydrogen, methyl, ethyl and -NO₂; 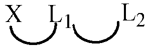
is of the formula (III): 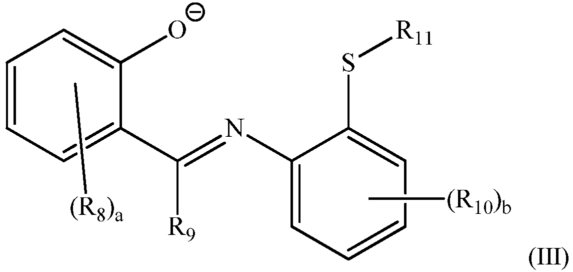 wherein:
   a and b are integers from 0 to 5;
   each R₈, R₉ and R₁₀ and R₁₁ may be the same or different and independently of the other selected from the group consisting of hydrogen, halogen, (C₁-C₁₆)alkyl, (C₁-C₁₆)alkoxy, (C₁-C₁₆)perhaloalkyl, (C₃-C₇)cycloalkyl, (C₂-C₁₆)alkenyl, (C₆-C₁₄)aryl, (C₆-C₁₄)perhaloaryl, (C₃-C₁₂)heterocyclyl, -OR₁₈, -NO₂, -COOH, -COOR₁₈,
   -CONR₁₈R₁₉, -SO₂NR₁₈R₁₉, -SO₂R₁₈, -CHO, -COR₁₈, wherein R₁₈ and R₁₉ are the same or different and each independently selected from the group consisting of (C₁-C₆)alkyl, (C₁-C₆)perhaloalkyl, (C₆-C₁₄)aryl, (C₆-C₁₄)perhaloaryl;
   L₃ is an N-heterocyclic carbene ligand of the formula (IVA) or (IVB): 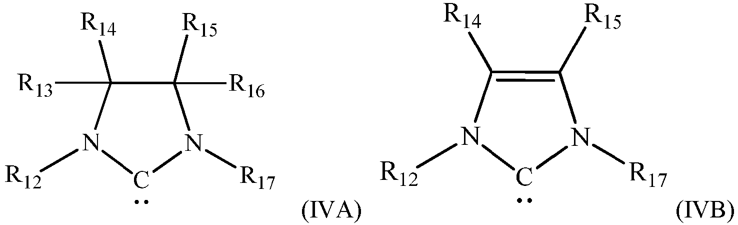 wherein:
      R₁₂ and R₁₇ are the same or different and each independently selected from the group consisting of (C₁-C₁₂)alkyl, (C₃-C₁₂)cycloalkyl, (C₂-C₁₂)alkenyl and (C₆-C₁₄)aryl, which is unsubstituted or substituted by (C₁-C₄)alkyl, (C₁-C₁₆)perhaloalkyl, (C₃-C₇)cycloalkyl or (C₄-C₂₀)alkylaryl;
      R₁₃, R₁₄, R₁₅ and R₁₆ are the same or different and each independently selected from the group consisting of hydrogen, (C₁-C₁₂)alkyl, (C₃-C₁₂)cycloalkyl, (C₂-C₁₂)alkenyl, (C₆-C₁₄)aryl, optionally substituted with at least one of (C₁-C₆)alkyl, (C₁-C₆)perhaloalkyl, (C₁-C₆)alkoxy or halogen; or
      R₁₃, R₁₄, R₁₅, R₁₆ may optionally join together with the carbon atoms to which they are attached to form a fused (C₄-C₈)carbocyclic ring unsubstituted or substituted by (C₁-C₄)alkyl, (C₁-C₁₆)perhaloalkyl, (C₃-C₇)cycloalkyl or (C₄-C₂₀)alkylaryl, , or to form a fused aromatic ring unsubstituted or substituted by (C₁-C₄)alkyl, (C₁-C₁₆)perhaloalkyl, (C₃-C₇)cycloalkyl and (C₄-C₂₀)alkylaryl,.

In yet some other embodiments, the compound of the formula (II) according to this invention is having: 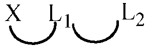 selected from the group consisting of:
a group of the formula (IIIA): 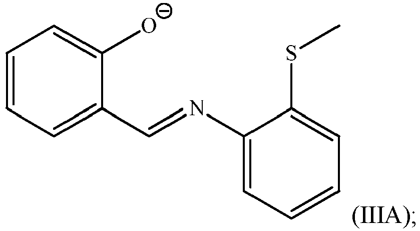
a group of the formula (IIIB): 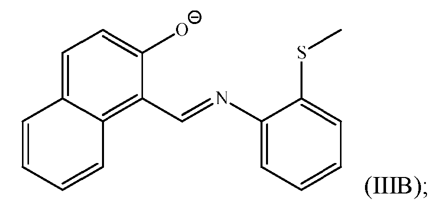
a group of the formula (IIIC): 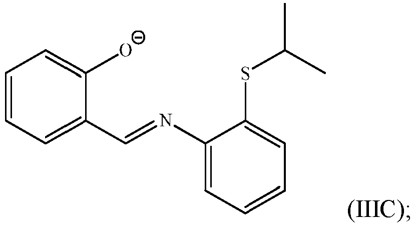
a group of the formula (IIID): 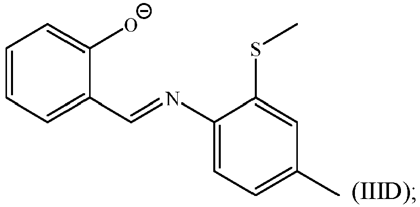
a group of the formula (IIIE): 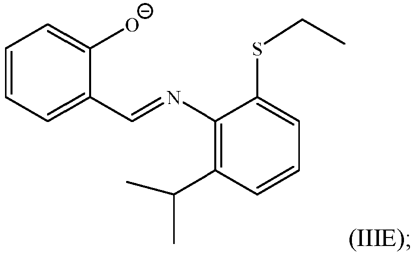
a group of the formula (IIIF): 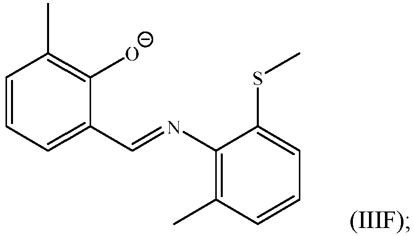 and
a group of the formula (IIIG):

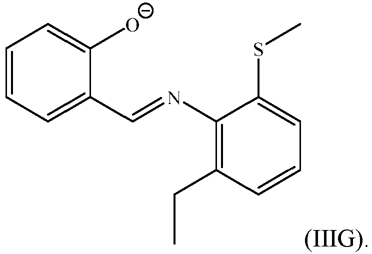

In some embodiments, any of the known N-heterocyclic carbene compounds can be used as L₃ ligands. Non-liming examples of such N-heterocyclic compounds are selected from the group consisting of: pyridine, 4-(N,N-dimethylamino)pyridine, 3-bromopyridine, piperidine, morpholine, pyridazine, pyrimidine, pyrazine, piperazine, 1,2,3-triazole, 1,3,4-triazole, 1,2,3-triazine as well as 1,2,4-triazine. Accordingly, in some embodiments, the compounds of the formulae (I) or (II) according to this invention are having:
L₃ selected from the group consisting of: 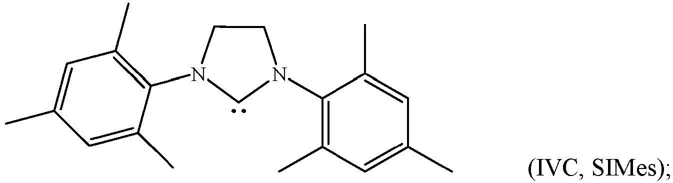 and

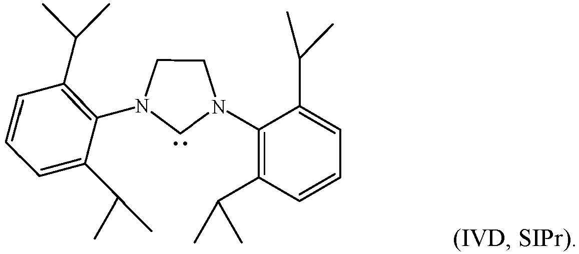

Representative non-limiting examples of the compound of the formula (II) may be enumerated as follows: 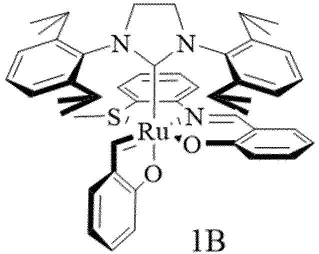 [1,3-Bis(2,6-diisopropylphenyl)-2-imidazolidinylidene]{2-[(E)-({2-[methylthio-κS]phenyl}imino-κN)methyl]phenoxido-κO}[2-(oxido-κO)benzylidene-κC]ruthenium(II); 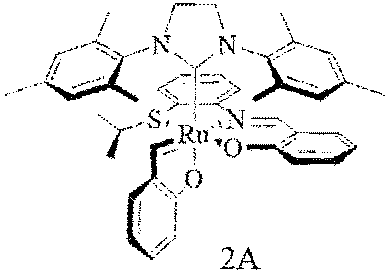 [1,3-Bis(2,4,6-trimethylphenyl)-2-imidazolidinylidene]{2-[(E)-({2-[isopropylthio-κS]phenyl}imino-κN)methyl]phenoxido-κO}[2-(oxido-κO)benzylidene-κC]ruthenium(II); 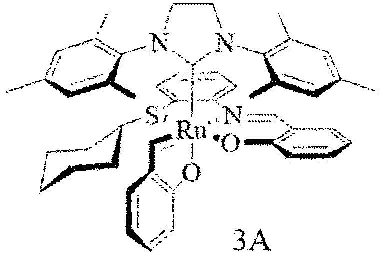 [1,3-Bis(2,4,6-trimethylphenyl)-2-imidazolidinylidene]{2-[(E)-({2-[cyclohexylthio-κS]phenyl}imino-κN)methyl]phenoxido-κO}[2-(oxido-κO)benzylidene-κC]ruthenium(II); and 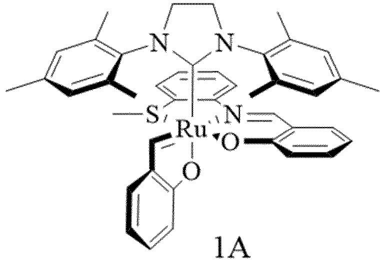 [1,3-Bis(2,4,6-trimethylphenyl)-2-imidazolidinylidene]{2-[(E)-({2-[methylthio-κS]phenyl}imino-κN)methyl]phenoxido-κO}[2-(oxido-κO)benzylidene-κC]ruthenium(II).

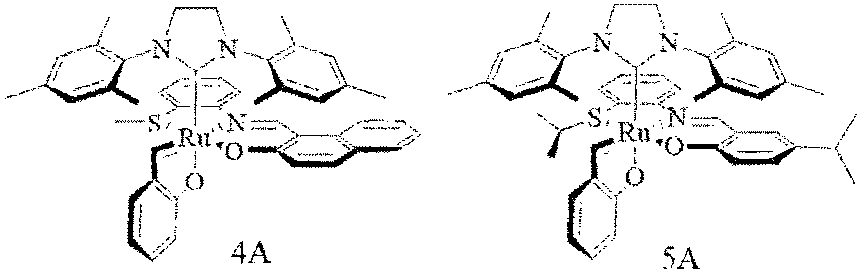

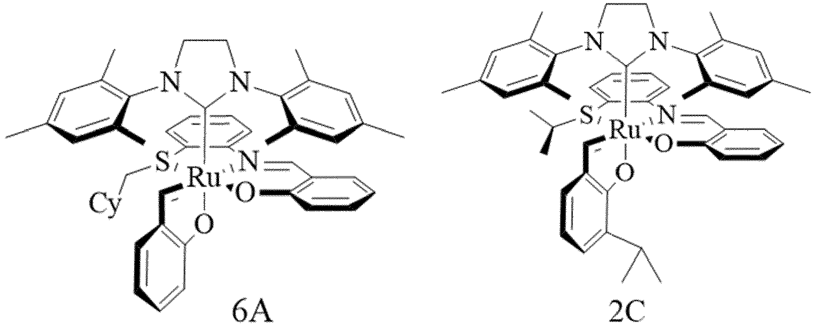

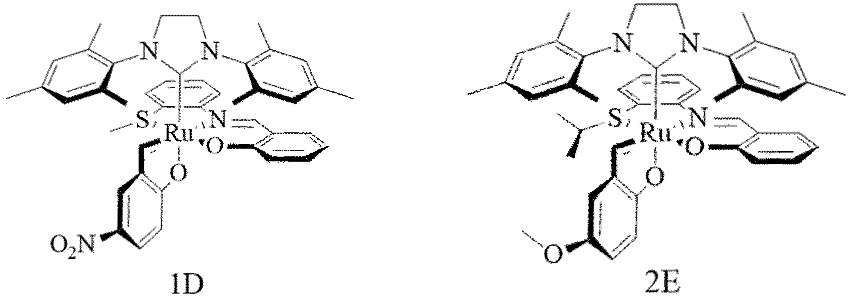

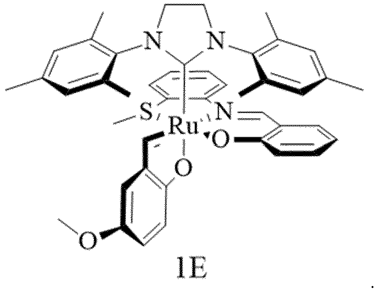

The invention concerns also a process for carrying out the metathesis reaction of olefins, wherein at least one olefin is contacted with a compound of the general formula (II) as a (pre)catalyst.

Generally, the metathesis reaction is carried out in an organic solvent. Any of the organic solvents that would allow such polymerization reaction to be carried out can be used. Non-limiting examples of such organic solvents include dichloromethane, dichloroethane, toluene, ethyl acetate and mixtures in any combination thereof.

In some embodiments, the metathesis reaction is carried out without any solvent. In some other embodiments, the metathesis reaction is carried out in the presence of a chemical activator. In general, the chemical activator is a Bronsted or Lewis acid or a haloderivative of alkane or silane. Non-limiting examples of such activators include hydrogen chloride, chlorotrimethylsilane or p-toluenesulfonic acid.

In some embodiments, the metathesis reaction is a ring-opening metathetic polymerization of dicyclopentadiene.

In yet some other embodiments, the (pre)catalyst of the general formula (I) is added in the solid form to dicyclopentadiene.

In one embodiment, the polymerization reaction is initiated by heating the mixture of dicyclopentadiene and the (pre)catalyst of the general formula (I) to a temperature of 30° C or higher.

In some embodiments, the metathesis reaction is carried out in the presence of an additive promoting formation of cross bonds.

Throughout the description of the invention and patent claims, if ppm (parts per million) units are used with relation to amount of substance, these are on a weight basis.

Since the inventors do not wish to be bound by any particular mechanism of catalysis, the "(pre)catalyst" term is used to indicate that the compound according to the invention may be either the catalyst itself or a precursor of the active species being the actual catalyst.

The definitions of groups not defined below should have the broadest meanings known in the art.

The term "optionally substituted" means that one or more hydrogen atoms of the group in question have been replaced with the specified groups, provided that such a substitution results in formation of a stable compound.

The term "halo" or "halogen" represents an element selected from F, Cl, Br, I.

The term "alkyl" concerns a saturated, straight-chain or branched-chain hydrocarbon substituent having the specified number of carbon atoms. The non-limiting examples of alkyls are: methyl, ethyl, propyl, isopropyl, butyl, sec-butyl, tert-butyl, pentyl.

The term "alkoxy" concerns the alkyl substituent, as defined above, bound via an oxygen atom.

The term "perhaloalkyl" represents the alkyl, as defined above, wherein all hydrogens have been replaced with halogen atoms, where the halogen atoms may be identical or different.

The term "cycloalkyl" concerns a saturated mono- or polycyclic hydrocarbon substituent having the specified number of carbon atoms. The non-limiting examples of a cycloalkyl substituent are cyclopropyl, cyclobutyl, cyclopentyl, cyclohexyl.

The term "alkenyl" concerns a non-cyclic, straight or branched hydrocarbon chain having the specified number of carbon atoms and containing at least one carbon-carbon double bond. The non-limiting examples of alkenyls are: vinyl, allyl, 1-butenyl, 2-butenyl.

The term "aryl" concerns an aromatic mono- or polycyclic hydrocarbon substituent having the specified number of carbon atoms. The non-limiting examples of aryl are: phenyl, mesityl, anthracenyl.

The term "heterocyclyl" concerns aromatic as well as non-aromatic cyclic substituents having the specified number of carbon atoms, wherein one or more carbon atoms have been replaced with a heteroatom such as nitrogen, phosphorus, sulfur, oxygen, provided that there are no two directly connected oxygen or sulfur atoms in the ring. Non-aromatic heterocyclyls can contain from 4 to 10 atoms in the ring, whereas aromatic heterocyclyls must have at least 5 atoms in the ring. The benzo-fused systems also belong to heterocyclyls. The non-limiting examples of non-aromatic heterocyclyls are: pyrrolidinyl, tetrahydrofuranyl, dihydrofuranyl, tetrahydrothienyl, tetrahydropyranyl, dihydropyranyl, tetrahydrothiopyranyl, piperidinyl, morpholinyl, thiomorpholinyl, 2-pyrrolinyl, indolinyl. The non-limiting examples of aromatic heterocyclyls are: pyridinyl, imidazolyl, pyrimidinyl, pyrazolyl, triazolyl, pyrazinyl, furyl, thienyl. The above-mentioned groups may be bound via a carbon atom or a nitrogen atom. For example, the substituent obtained by binding pyrrole may be either pyrrol-1-yl (N-bound) or pyrrol-3-yl (C-bound).

The term "neutral ligand" concerns a substituent having no electrical charge, capable of coordinating to a ruthenium atom. The non-limiting examples of such ligands are: N-heterocyclic carbene ligands, amines, imines, phosphines and oxides thereof, alkyl and aryl phosphites and phosphates, ethers, alkyl and aryl sulfides, coordinated hydrocarbons, haloalkanes and haloarenes. The term "neutral ligand" encompasses also N-heterocyclic compounds; their non-limiting examples are: pyridine, 4-(N,N-dimethylamino)pyridine (DMAP), 3-bromopyridine, piperidine, morpholine, pyridazine, pyrimidine, pyrazine, piperazine, 1,2,3-triazole, 1,3,4-triazole, 1,2,3-triazine and 1,2,4-triazine.

The term "anionic ligand" concerns the substituent capable to co-ordination with a metal center, bearing an electrical charge capable to compensate the charge of the metal center, wherein such a compensation may be complete or partial. The non-limiting examples of anionic ligands are: fluoride, chloride, bromide or iodide anions, carboxylic acid anions, alcohol and phenol anions, thiol and thiophenol anions, (organo)sulfuric and (organo)phosphoric acid anions as well as anions of esters thereof.

The term "carbene" concerns a molecule containing a neutral carbon atom having the valence number of 2 and two non-paired valence electrons. The term "carbene" encompasses also carbene analogues, wherein the carbon atom is replaced with another chemical element such as: boron, silicon, nitrogen, phosphorus, sulfur. The term "carbene" relates particularly to N-heterocyclic carbene (NHC) ligands. The non-limiting examples of the NHC ligands are: 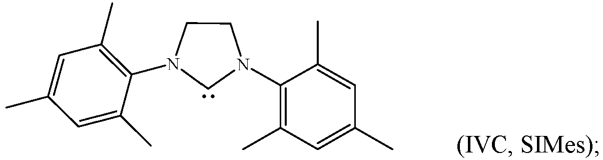 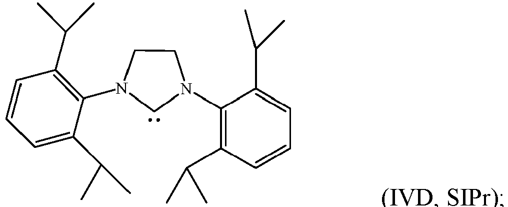 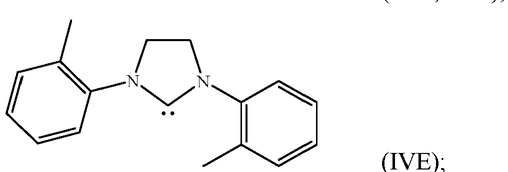 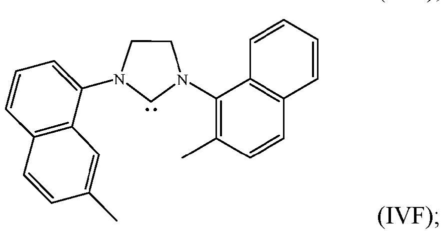 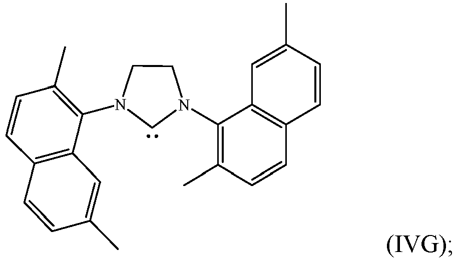 and

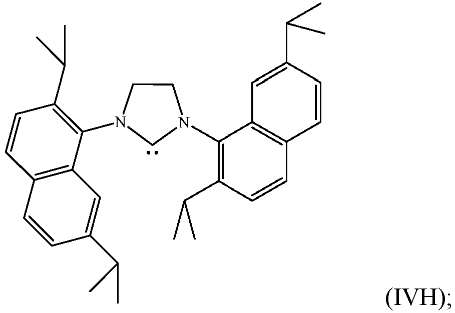

The expression "stereoisomers" as used herein is a general term used for all isomers of the individual molecules that differ only in the orientation of their atoms in space. Typically it includes mirror image isomers that are usually formed due to at least one asymmetric center, (enantiomers). Where the compounds according to the invention possess two or more asymmetric centers, they may additionally exist as diastereomers, also certain individual molecules may exist as geometric isomers (cis/trans). Similarly, certain compounds of this invention may exist in a mixture of two or more structurally distinct forms that are in rapid equilibrium, commonly known as tautomers. Representative examples of tautomers include keto-enol tautomers, phenol-keto tautomers, nitroso-oxime tautomers, imine-enamine tautomers, etc. It is to be understood that all such isomers and mixtures thereof in any proportion are encompassed within the scope of the present invention.

The non-limiting examples of preferred agents promoting formation of cross bonds are tert-butyl peroxide, di-tert-butyl peroxide, and also mixtures thereof.

The following examples describe the procedures used for the preparation of the compounds of this invention and their use in olefin metathesis. The following examples are only intended to illustrate the invention and to explain its particular aspects. The activity of the catalyst (1B) according to the invention was compared to the LatMetSIMes3D3, which structure is presented below:

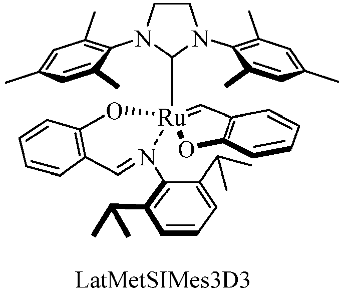

DCPD used in the following Examples was purchased from commercial sources (Ultrene 99-6 from Cymetech) and contained six mass percent of tricyclopentadiene (TCPD), triphenylphosphine, solution of lithium bis(trimethylsilyl)amide, solution of potassium tert-pentoxide, hydrogen chloride solution in 1,4-dioxane were purchased from commercially sources. All reactions were carried out under argon. The toluene was washed with citric acid, water, dried with 4Å molecular sieves and deoxidized with argon. The THF was dried with 4Å molecular sieves and deoxidized with argon.

### Example 1

### Step 1:

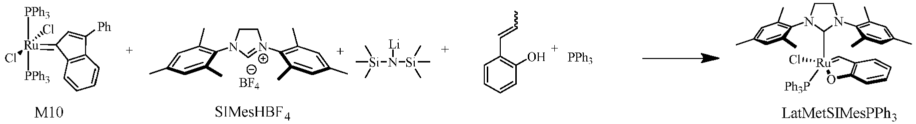

A toluene solution of lithium bis(trimethylsilyl)amide (1 M, 12 mL, 1.1 eq.) was added to a suspension of SIMesHBF₄ (5.1 g, 1.15 eq.) in toluene (82 mL). The resulting mixture was stirred at room temperature for 30 min and then placed in an oil bath heated to a temperature of 80 °C. After 10 minutes a compound of formula M10 (10 g, 11.3 mmol, 1 eq.) was added and the mixture was stirred for 10 minutes. Next, (*E*/*Z*)-2-(prop-1-en-1-yl)phenol (2.27 g, 1.5 eq.) was added and after additional 30 minutes triphenylphosphine was added (1.48 g, 0.5 eq.). The reaction mixture was stirred at 80 °C for 90 minutes, then cooled down to room temperature and filtered through a short pad of silica gel. The silica gel pad was washed with toluene. The crude product was purified by crystallization and recrystallization from dichloromethane/n-heptane mixture, green solid, 4.2 g, 46% yield.

### Step 2:

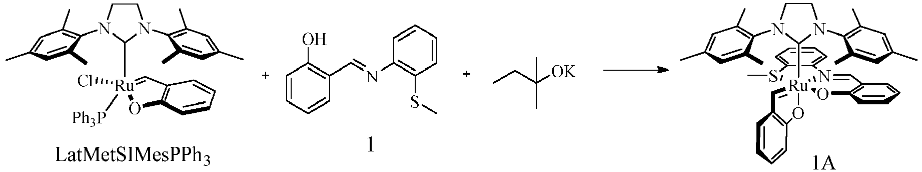

A toluene solution of potassium *tert*-pentoxide (1.7 M, 1.74 mL, 1.2 eq.) was added to a solution of imine 1 (0.72 g, 1.2 eq.) in tetrahydrofuran (23 mL) and the resulting mixture was stirred at room temperature for 30 min. After that, LatMetSIMesPPh₃ (2 g, 2.47 mmol, 1 eq.) was added and the reaction was stirred at 41 °C for 45 min. The reaction mixture was filtered through a short pad of celite. The celite pad was washed with THF. The solvents were evaporated to dryness and the crude product was crystallized from a dichloromethane/methanol mixture, black crystals, 0.95 g, 51% yield. The compound was characterized by ¹H and ¹³C NMR:
¹H NMR (CD₂Cl₂, 5.32 ppm; 600 MHz): *δ* [ppm] = 13.94 (s, 1H), 8.46 (s, 1H), 7.50 (d, 1H), 7.36 (ddd, 1H), 7.33 (dd, 1H), 7.18-7.14 (m, 1H), 7.13 (dd, 1H), 7.05 (ddd, 1H), 6.94 (ddd, 1H), 6.77 (s, 2H), 6.64 (s, 2H), 6.54-6.47 (m, 2H), 6.44 (ddd, 1H), 6.26 (d, 1H), 6.20 (ddd, 1H), 3.87-3.75 (m, 4H), 2.30 (s, 6H), 2.21 (s, 6H), 2.07 (s, 6H), 1.23 (s, 3H).
¹³C NMR (CD₂Cl₂, 54.00 ppm; 150 MHz): *δ* [ppm] = 297.4, 215.4, 178.2, 170.7, 158.2, 152.9, 151.7, 139.6, 138.0, 137.8, 137.5, 136.8, 133.2, 132.9, 130.7, 129.6, 129.4, 129.3, 126.1, 125.5, 124.4, 122.4, 118.6, 118.2, 113.4, 112.2, 52.8, 23.1, 21.4, 18.7, 18.6.

### Example 2

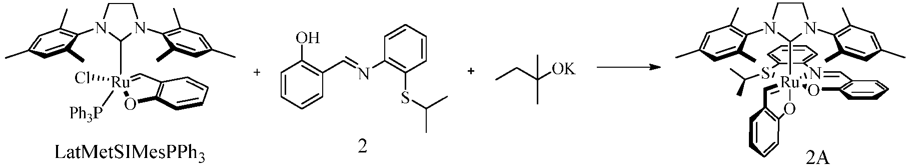

A toluene solution of potassium *tert*-pentoxide (1.7 M, 5.23 mL, 1.2 eq.) was added to a solution of imine 2 (2.41 g, 1.2 eq.) in tetrahydrofuran (68.8 mL) and the resulting mixture was stirred at room temperature for 30 minutes. After that, LatMetSIMesPPh₃ (6 g, 7.4 mmol, 1 eq.) was added and the reaction was stirred at 41 °C for 1 hour. The reaction mixture was filtered through a short pad of celite. The celite pad was washed with THF. The solvents were evaporated to dryness and the crude product was crystallized from a dichloromethane/methanol mixture, black crystals, 4.76 g, 82% yield. The compound was characterized by ¹H and ¹³C NMR:
¹H NMR (CD₂Cl₂, 5.32 ppm; 600 MHz): *δ* [ppm] = 13.89 (s, 1H), 7.59 (s, 1H), 7.29 (dd, 1H), 7.22 (ddd, 1H), 7.00-6.90 (m, 4H), 6.81 (s, 2H), 6.77 (dd, 1H), 6.68 (ddd, 1H), 6.52 (s, 2H), 6.37-6.31 (m, 2H), 6.09 (d, 1H), 6.01 (ddd, 1H), 3.77-3.67 (m, 2H), 3.60-3.50 (m, 2H), 2.37 (s, 6H), 2.35 (s, 6H), 2.13 (s, 6H), 0.87 (d, 3H), 0.67 (hept, 1H), 0.26 (d, 3H).
¹³C NMR (CD₂Cl₂, 54.00 ppm; 150 MHz): *δ* [ppm] = 292.0, 207.8, 181.3, 172.7, 161.7, 158.4, 152.1, 138.1, 137.9, 137.8, 137.3, 135.7, 135.6, 133.8, 131.8, 129.5, 129.4, 129.2, 128.5, 125.3, 124.8, 123.7, 122.6, 118.7, 115.8, 111.9, 111.6, 52.8, 41.9, 23.5, 21.3, 19.2, 18.7, 18.4.

### Example 3

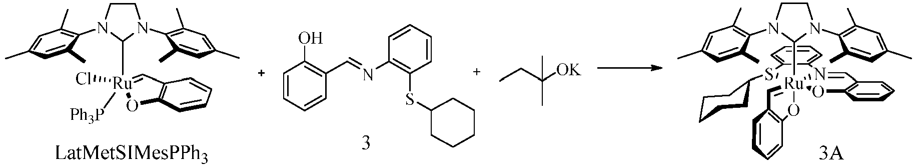

A toluene solution of potassium *tert*-pentoxide (1.7 M, 0.44 mL, 1.2 eq.) was added to a solution of imine 3 (0.23 g, 1.2 eq.) in tetrahydrofuran (11.9 mL) and the resulting mixture was stirred at room temperature for 30 minutes. After that, LatMetSIMesPPh₃ (0.5 g, 0.62 mmol, 1 eq.) was added and the reaction was stirred at 41 °C for 1 hour. The reaction mixture was filtered through a short pad of celite. The celite pad was washed with THF. The solvents were evaporated to dryness and the crude product was crystallized from a dichloromethane/methanol mixture, black crystals, 0.23 g, 45% yield. The compound was characterized by ¹H and ¹³C NMR:
¹H NMR (CD₂Cl₂, 5.32 ppm; 600 MHz): *δ* [ppm] = 13.86 (s, 1H), 7.59 (s, 1H), 7.28 (dd, 1H), 7.21 (ddd, 1H), 6.98-6.88 (m, 4H), 6.78 (s, 2H), 6.75 (dd, 1H), 6.67 (ddd, 1H), 6.53 (s, 2H), 6.34 (ddd, 1H), 6.31 (dd, 1H), 6.08 (d, 1H), 6.00 (ddd, 1H), 3.75-3.65 (m, 2H), 3.58-3.48 (m, 2H), 2.36 (s, 6H), 2.35 (s, 6H), 2.15 (s, 6H), 1.71-1.63 (m, 1H), 1.62-1.55 (m, 1H), 1.55-1.48 (m, 1H), 1.34-1.23 (m, 1H), 1.22-1.12 (m, 1H), 1.03 (qt, 1H), 0.88 (qd, 1H), 0.77 (qt, 1H), 0.53-0.41 (m, 2H), -0.25 (qd, 1H).
¹³C NMR (CD₂Cl₂, 54.00 ppm; 150 MHz): *δ* [ppm] = 291.7, 208.0, 181.2, 172.7, 161.5, 158.4, 152.1, 138.2, 137.9, 137.7, 137.3, 136.1, 135.5, 133.7, 131.8, 129.4 (2 x C), 129.0, 128.8, 125.3, 124.6, 123.7, 122.6, 118.6, 115.8, 111.8, 111.6, 52.9, 49.8, 33.9, 29.5, 27.2, 27.0, 25.7, 21.5, 18.7, 18.4.

### Example 4

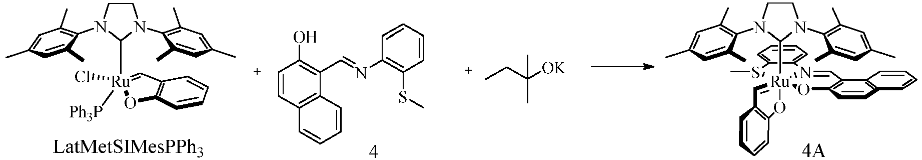

A toluene solution of potassium *tert*-pentoxide (1.7 M, 0.44 mL, 1.2 eq.) was added to a solution of imine 4 (0.22 g, 1.2 eq.) in tetrahydrofuran (13.9 mL) and the resulting mixture was stirred at room temperature for 30 minutes. After that, LatMetSIMesPPh₃ (0.5 g, 0.62 mmol, 1 eq.) was added and the reaction was stirred at 41 °C for 1 hour. The reaction mixture was filtered through a short pad of celite. The celite pad was washed with THF. The solvents were evaporated to dryness and the crude product was crystallized from a dichloromethane/methanol mixture, brown powder, 0.20 g, 40% yield. The compound was characterized by ¹H and ¹³C NMR:
¹H NMR (CD₂Cl₂, 5.32 ppm; 600 MHz): *δ* [ppm] = 14.11 (s, 1H), 9.52 (s, 1H), 8.07 (d, 1H), 7.67 (d, 1H), 7.59 (d, 1H), 7.50-7.38 (m, 3H), 7.32 (dd, 1H), 7.19 (ddd, 1H), 7.15 (ddd, 1H), 6.94 (ddd, 1H), 6.72-6.61 (m, 5H), 6.58 (dd, 1H), 6.26 (d, 1H), 6.21 (ddd, 1H), 3.87-3.74 (m, 4H), 2.21 (s, 6H), 2.19 (s, 6H), 2.08 (s, 6H), 1.26 (s, 3H).
¹³C NMR (CD₂Cl₂, 54.00 ppm; 150 MHz): *δ* [ppm] = 299.1, 214.9, 178.4, 171.2, 153.0, 152.9, 151.2, 139.5, 137.9, 137.7, 137.3, 136.8, 133.4, 133.0, 132.6, 130.7, 129.5, 129.4, 129.3 (2 x C), 127.8, 127.3, 125.7, 125.2, 121.7, 119.4, 118.8, 118.5, 112.2, 111.4, 52.7, 22.8, 21.3, 18.7, 18.6.

### Example 5

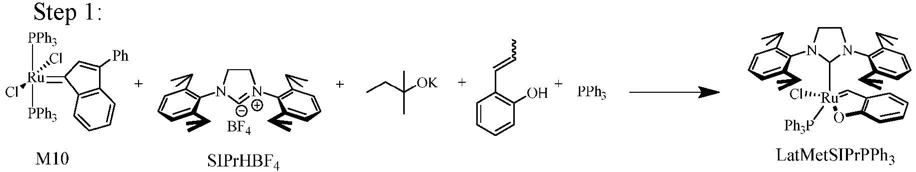

A toluene solution of potassium *tert*-pentoxide (1.7 M, 139 mL, 1.05 eq.) was added to a suspension of SIPrHBF₄ (113 g, 1.05 eq.) in toluene (2050 mL). The resulting mixture was stirred at room temperature for 30 minutes and then placed in an oil bath heated to a temperature of 85 °C. After 20 minutes M10 (200 g, 226 mmol, 1 eq.) was added followed by an addition of toluene (50 mL). The mixture was stirred for 30 minutes. After that, (*E*/*Z*)-2-(prop-1-en-1-yl)phenol (45.4 g, 1.5 eq.) in toluene (50 mL) was added followed by an addition of triphenylphosphine (59.2 g, 1 eq.). The reaction mixture was stirred at 85 °C for 90 minutes, then cooled down to room temperature and filtered through a short pad of silica. The silica gel pad was washed with toluene. The solvents were evaporated to dryness and the crude product was crystallized from a dichloromethane/methanol mixture, 38.6 g, 19% yield.

### Step 2:

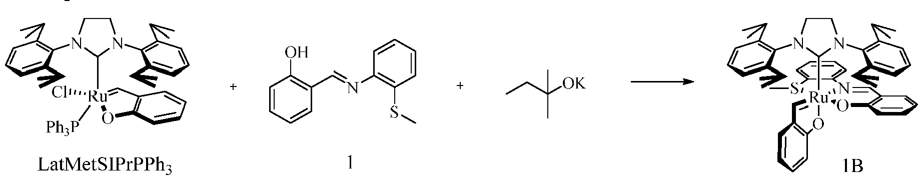

A toluene solution of potassium *tert*-pentoxide (1.7 M, 22 mL, 1.21 eq.) was added to a solution of imine 1 (9.18 g, 1.22 eq.) in tetrahydrofuran (288 mL) and the resulting mixture was stirred at room temperature for 30 minutes. After that, LatMetSIPrPPh₃ (27.73 g, 31 mmol, 1 eq.) was added and the reaction was stirred at 61 °C for one hour, then cooled down to room temperature and filtered through a short pad of celite. The celite pad was washed with tetrahydrofuran and dichloromethane. The solvents were evaporated to dryness and the crude product was crystallized from a dichloromethane/*n*-heptane mixture yielded 1B as a mixture of diastereomers, brown powder, 27.05 g, 104% yield of crude product. The brown powder was dissolved in dichloromethane (500 mL). Then, methanol (150 mL) was added portion-wise over a period of 5 hours to a stirring brown solution. The resulting solution was stirred at room temperature for one hour and filtered through a filter paper. Dichloromethane was slowly evaporated to yield 1B as a single diastereomer, black crystals. The product was recrystallized from a dichloromethane/methanol mixture, black crystals, 20.82 g, 80% yield. The compound was characterized by ¹H and ¹³C NMR:
¹H NMR (CD₂Cl₂, 5.32 ppm; 600 MHz): *δ* [ppm] = 15.12 (s, 1H), 8.55 (s, 1H), 7.59 (d, 1H), 7.36 (ddd, 1H), 7.15-7.06 (m, 3H), 7.06-6.98 (m, 4H), 6.96 (dd, 1H), 6.94-6.88 (m, 3H), 6.65 (ddd, 1H), 6.36 (d, 1H), 6.29 (ddd, 1H), 6.24 (ddd, 1H), 5.71 (d, 1H), 4.16-4.06 (m, 2H), 3.93-3.82 (m, 2H), 3.60 (hept, 2H), 3.26 (hept, 2H), 1.21 (d, 6H), 1.17 (d, 6H), 1.11 (s, 3H), 1.00 (d, 6H), 0.67 (d, 6H).
¹³C NMR (CD₂Cl₂, 54.00 ppm; 150 MHz): *δ* [ppm] = 294.1, 215.5, 179.6, 171.6, 161.6, 153.7, 152.2, 146.8, 146.7, 141.1, 135.8, 133.0, 132.5, 132.2, 132.1, 130.8, 128.5, 126.2, 125.3, 124.8, 124.6, 124.3, 122.1, 118.9, 117.9, 113.1, 112.3, 56.0, 28.9, 28.7, 26.6, 26.5, 23.5, 23.2, 21.1.

### Example 6

A compound of this invention was used to show the shelf life stability of the compounds of this invention when compared with an organoruthenium compound disclosed in the art as shown in the Comparative Example 1 below.

A compound of Example 5, compound 1B, was used in this study: 1B (14.93 mg, 40 mol ppm) was dissolved in 60 mL of Ultrene 99-6, which was designated as formulation A. The resulting solution was stored under argon at room temperature. The shelf life of formulation A was monitored in a test reaction with a freshly prepared formulation B containing HCl (200 mol ppm) once every two weeks.

### A test procedure:

A solution of hydrogen chloride (4 M solution in 1,4-dioxane, 1.85 µL, 200 mol ppm) in 5 mL of Ultrene 99-6 was prepared, and was designated as formulation B. The formulation B as formed was added to 5 mL of freshly prepared formulation A (1.244 mg, 40 mol ppm of 1B). The final, reactive formulation contained 20 mol ppm of 1B and 100 mol ppm of HCl. The results are summarized in Table 1.

**Table 1**

| Time (seconds) | Observations |
|---|---|
| 0 | Formulations A and B were mixed |
| 6 | Gelation point |
| 13 | Smoke time |
| | Peak exotherm Tₘₐₓ = 202 °C |

The formulation A was then stored at room temperature for 30 weeks. After which time a freshly prepared formulation B (5 mL of Ultrene 99-6 with hydrogen chloride; 4 M solution in 1,4-dioxane, 1.85 µL, 200 mol ppm) was added to 5 mL of formulation A (1.244 mg, 40 mol ppm of 1B). The final, reactive formulation contained 20 mol ppm of 1B and 100 mol ppm of HCl. The results are summarized in Table 2.

**Table 2**

| Time (seconds) | Observations |
|---|---|
| 0 | Formulations A and B were mixed |
| 2 | Gelation point |
| 6 | Smoke time |
| | Peak exotherm Tₘₐₓ = 203 °C |

It is evident from the data presented in Tables 1 and 2, the catalytic activity of the compound of this invention, i.e., 1B is not negatively affected even after storing at room temperature for 30 weeks. In fact, the solution of formulation A is even more active after 30 weeks than it was immediately after it was prepared.

### Comparative Example 1

LatMetSIMes3D3 (14.11 mg, 40 mol ppm) was dissolved in 60 mL of Ultrene 99-6 giving formulation A. The resulting solution was stored under argon at room temperature. The shelf life of formulation A was monitored in a test reaction with a freshly prepared formulation B containing HCl (200 mol ppm) once every two weeks.

### A test procedure:

5 mL of Ultrene 99-6 with hydrogen chloride (4 M solution in 1,4-dioxane, 1.85 µL, 200 mol ppm) was prepared and designated as formulation B. Formulation B was added to 5 mL of formulation A (1.176 mg, 40 mol ppm of 1B). The final, reactive formulation contained 20 mol ppm of LatMetSIMes3D3 and 100 mol ppm of HCl. The results are summarized in Table 3.

**Table 3**

| Time [s] | Observations |
|---|---|
| 0 | Formulations A and B were mixed |
| 9 | Gelation point |
| 15 | Smoke time |
| | Peak exotherm Tₘₐₓ = 212 °C |

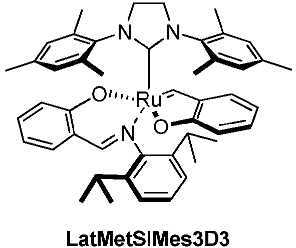

5 mL of fresh formulation B was prepared as described above. Formulation B was added to formulation A (5 mL, 40 mol ppm of LatMetSIMes3D3) which was stored for 2 weeks. The final, reactive concentration contained 20 mol ppm of LatMetSIMes3D3 and 100 mol ppm of HCl. The reactive formulation was gelled and no peak exotherm was observed which was the result of partial catalyst decomposition.

### Example 7

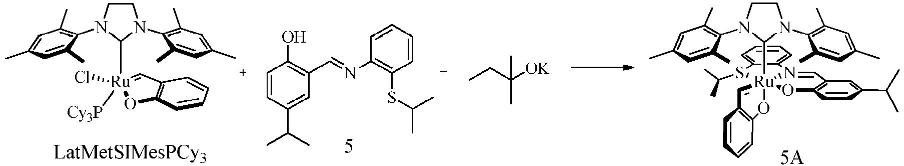

A toluene solution of potassium *tert*-pentoxide (1.7 M, 4.26 mL, 1.2 eq.) was added to a solution of imine 5 (2.27 g, 1.2 eq.) in 1,4-dioxane (56.0 mL) and the resulting mixture was stirred at room temperature for 30 minutes. After that, LatMetSIMesPCy₃ (5 g, 6.0 mmol, 1 eq.) was added and the reaction was stirred at 95 °C for 2 hours. The reaction mixture was cooled down to room temperature and filtered through a short pad of celite. The celite pad was washed with 1,4-dioxane and dichloromethane. The solvents were evaporated to dryness and the crude product was crystallized from a dichloromethane/*n-*heptane mixture yielded 5A as a mixture of diastereomers, brown powder, 4.16 g, 84% yield of crude product. The product was recrystallized from a dichloromethane/methanol mixture yielded pure 5A as a mixture of diastereomers, deep brown or black crystals 3.05 g, 61% yield. The pure mixture of diastereomers was dissolved in dichloromethane (30 mL). Then, methanol (45 mL) was added slowly. The resulting solution was stirred at room temperature overnight. The solvents were evaporated slowly to dryness yielded 5A as a single diastereomer, deep brown or black crystals, 3.01 g, 61% yield. The compound was characterized by ¹H and ¹³C NMR:
¹H NMR (CD₂Cl₂, 5.32 ppm; 600 MHz): *δ* [ppm] = 14.07 (s, 1H), 8.36 (s, 1H), 7.62 (d, 1H), 7.45-7.41 (m, 1H), 7.40 (d, 1H), 7.19-7.14 (m, 1H), 6.97-6.89 (m, 2H), 6.84 (d, 1H), 6.76-6.71 (m, 2H), 6.69-6.62 (m, 3H), 6.38 (d, 1H), 6.26 (d, 1H), 6.24-6.19 (m, 1H), 3.89-3.67 (m, 4H), 2.79 (hept, 1H), 2.28 (s, 6H), 2.19 (s, 6H), 2.07-1.99 (m, 7H), 1.28-1.22 (m, 6H), 0.49 (d, 3H), 0.30 (d, 3H).
¹³C NMR (CD₂Cl₂, 54.00 ppm; 150 MHz): *δ* [ppm] = 296.1, 214.7, 177.9, 169.5, 157.6, 152.9, 152.8, 139.5, 138.1, 137.7, 137.6, 133.1, 133.1, 132.4, 132.3, 131.9, 130.5, 129.6, 129.4, 128.9, 125.5, 125.1, 124.1, 121.8, 118.6, 118.2, 112.0, 52.6, 45.2, 33.3, 24.6, 24.4, 22.4, 21.4, 20.6, 19.0, 18.7.

### Example 8

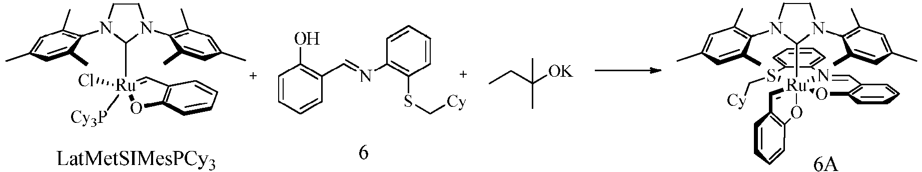

A toluene solution of potassium *tert*-pentoxide (1.7 M, 4.26 mL, 1.2 eq.) was added to a solution of imine 6 (2.36 g, 1.2 eq.) in 1,4-dioxane (56.0 mL) and the resulting mixture was stirred at room temperature for 30 minutes. After that, LatMetSIMesPCy₃ (5 g, 6.0 mmol, 1 eq.) was added and the reaction was stirred at 95 °C for 2 hours. The reaction mixture was cooled down to room temperature and filtered through a short pad of celite. The celite pad was washed with 1,4-dioxane and dichloromethane. The solvents were evaporated to dryness and the crude product was crystallized from a dichloromethane/n-heptane mixture yielded 6A as a mixture of diastereomers, brown powder, 4.74 g, 94% yield of crude product. The brown powder was dissolved in a mixture of dichloromethane (30 mL) and methanol (150 mL). The resulting solution was stirred at room temperature overnight. Dichloromethane was slowly evaporated to yield 6A as a single diastereomer, black crystals, 3.18 g, 63% yield. The compound was characterized by ¹H and ¹³C NMR:
¹H NMR (CD₂Cl₂, 5.32 ppm; 600 MHz): *δ* [ppm] = 14.14 (s, 1H), 8.41 (s, 1H), 7.39 (ddd, 1H), 7.33 (dd, 1H), 7.22-7.14 (m, 1H), 7.08-6.99 (m, 2H), 6.95 (ddd, 1H), 6.72 (d, 4H), 6.61 (dd, 1H), 6.46 (d, 1H), 6.40 (ddd, 1H), 6.30 (d, 1H), 6.23 (ddd, 1H), 3.88-3.70 (m, 4H), 2.29 (s, 6H), 2.19 (s, 6H), 2.09 (s, 6H), 1.76-1.58 (m, 1H), 1.56-1.44 (m, 2H), 1.38-1.28 (m, 1H), 1.03 (qt, 1H), 0.94 (qt, 1H), 0.79 (qt, 1H), 0.60 (qd, 1H), 0.40-0.31 (m, 1H), 0.26-0.08 (m, 2H).
¹³C NMR (CD₂Cl₂, 54.00 ppm; 150 MHz): *δ* [ppm] = 297.0, 296.9, 214.7, 178.2, 170.5, 158.3, 153.0, 152.0, 139.5, 138.0, 137.6, 136.8, 133.2, 133.0, 132.8, 131.5, 129.5, 129.2, 129.1, 126.1, 125.8, 124.3, 122.2, 118.8, 118.2, 113.2, 112.5, 52.6, 48.5, 36.4, 32.8, 32.2, 26.8, 26.8, 26.5, 21.4, 18.8, 18.7.

### Example 9

The structure of complex 1B, as illustrated below, was determined by X-ray crystallographic analysis. Crystals of 1B for this analysis were grown from a dichloromethane/*n*-heptane solution.

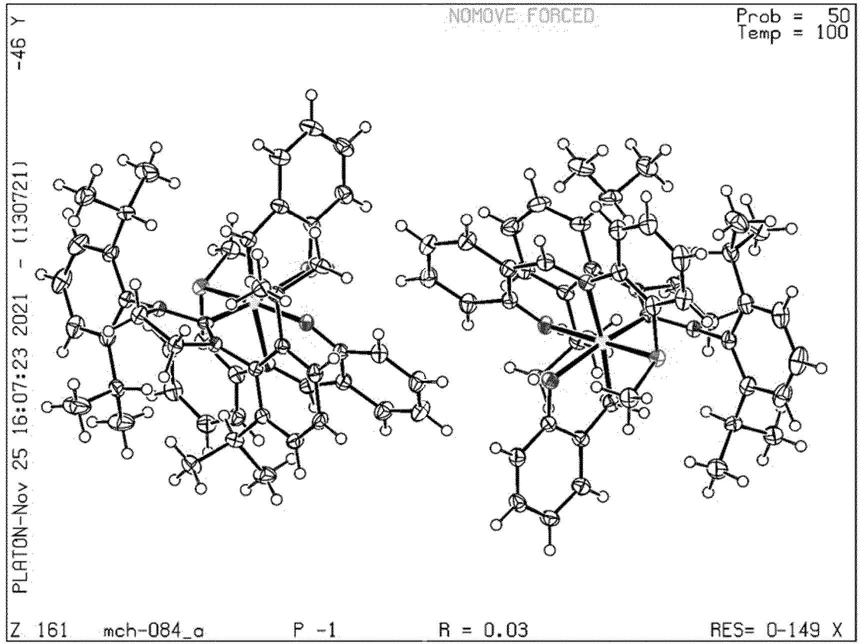

| | | | | |
|---|---|---|---|---|
| Bond precision: | C-C = 0.0032 A | Wavelength=1.54184 | | |
| Cell: | a=12.5757(2) | b=14.3682(2) | | c=23.1131(3) |
| | alpha=84.759(1) | beta=84.390(1) | | gamma=81.729(1) |
| Temperature: | 100 K | | | |

| | Calculated | | Reported | |
|---|---|---|---|---|
| Volume | 4100.39(10) | | 4100.39(10) | |
| Space group | P -1 | | P -1 | |
| Hall group | -P 1 | | -P 1 | |
| Moiety formula | C48 H55 N3 O2 Ru S, C48 H56 N3 O2 Ru S | | C48 H55.50 N3 O2 Ru S | |
| Sum formula | C96 H111 N6 O4 Ru2 S2 | | C48 H55.50 N3 O2 Ru S | |
| Mr | 1679.17 | | 839.58 | |
| Dx,g cm-3 | 1.360 | | 1.360 | |
| Z | 2 | | 4 | |
| Mu (mm-1) | 3.897 | | 3.897 | |
| F000 | 1762.0 | | 1762.0 | |
| F000' | 1767.63 | | | |
| h, k, lmax | 15, 18, 29 | | 15, 18, 28 | |
| Nref | 17513 | | 16307 | |
| Tmin,Tmax | 0.795, 0.947 | | 0.599, 1.000 | |
| Tmin' | 0.749 | | | |
| Correction method= # | Reported T Limits: | Tmin=0.599 Tmax=1.000 | | |
| AbsCorr = MULTI-SCAN | | | | |
| Data completeness= 0.931 | | Theta(max)= 77.936 | | |
| R(reflections)= 0.0332( 15104) | | wR2(reflections)= 0.0975( 16307) | | |
| S = 1.086 | Npar= 991 | | | |
| | | | | |

### Example 10

### Step 1:

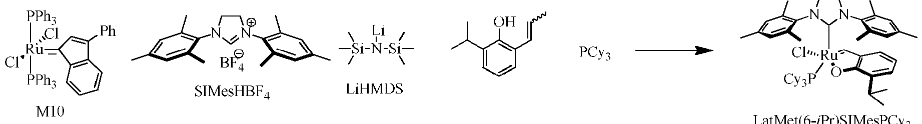

SIMesHBF₄ (7.81 g, 19.8 mmol, 1.1 eq.) was placed under argon in a round bottomed flask. Toluene (160 mL) was added and the resulted suspension was heated up to 80 °C. Next, LiHMDS (1 M in toluene, 19.8 mL, 1.1 eq.) was added and the mixture was stirred for 3 minutes before an addition of M10 (15.96 g, 18.0 mmol, 1 eq.). After 15 min full conversion of M10 was observed on TLC plate (AcOEt/*c*-C₆H₁₂, 1:9, v/v). The temperature was raised to 110 °C and (*E*/*Z*)-2-(prop-1-en-1-yl)-6-isopropylphenol (7.29 g, 36.0 mmol, 2 eq.) was added. The resulted mixture was stirred for 20 min before tricyclohexylphosphine (5.55 g, 19.8 mmol, 1.1 eq.) was added. The stirring was continued for additional 2.5 hours. After that time, the reaction mixture was cooled down to room temperature and was filtered through a short pad of celite. The filtrate was concentrated and the crude product was purified by column chromatography (*c*-C₆H₁₂ to *c-*C₆H₁₂/AcOEt, 98:2, v/v). Solvents were removed and the crude product was crystalized two times from a dichloromethane/methanol mixture, green solid, 5.26 g, 33% yield. The compound was characterized by ¹H, ³¹P and ¹³C NMR:
¹H NMR (CD₂Cl₂, 5.32 ppm; 600 MHz): *δ* [ppm] = 15.72 (d, *J* = 1.3 Hz, 1H), 7.09 (bs, 1H), 7.01 (bs, 1H), 6.67 (bs, 1H), 6.93-6.89 (dd, *J* = 7.0, 1.6 Hz, 1H), 6.27 (dd, *J* = 7.7, 1.7 Hz, 2H), 6.22-6.20 (m, 1H), 3.98 (ddd, *J =* 11.5, 9.0, 7.2 Hz, 1H), 3.81-3.68 (m, 3H), 3.60 (ddd, *J =* 10.4, 9.2, 7.2 Hz, 1H), 2.67 (s, 3H), 2.53 (d, *J =* 9.1 Hz, 6H), 2.35 (s, 3H), 2.28 (s, 3H), 1.67-1.64 (m, 3H), 1.62-1.54 (m, 9H), 1.44-1.41 (m, 3H), 1.19-1.16 (m, 9H), 1.10-1.00 (m, 9H), 0.97-0.94 (m, 3H), 0.84 (qt, *J =* 12.4, 2.8 Hz, 3H), 0.67 (qt, *J =* 12.8, 3.0 Hz, 3H).
³¹P NMR (CD₂Cl₂, 240 MHz): *δ* [ppm] = 28.05.
¹³C NMR (CD₂Cl₂, 54.00 ppm; 150 MHz): *δ* [ppm] = 281.4, 222.5, 222.0, 178.1, 147.9, 139.9, 139.5, 138.9, 137.7, 137.6, 137.5, 137.1, 135.9, 134.7, 130.4, 130.3, 129.9, 129.1, 125.8, 120.5, 111.7, 51.8, 51.7, 32.3, 32.2, 29.4, 29.1, 28.2, 28.2, 28.1, 28.1, 27.1, 25.8, 24.0, 23.8, 21.7, 21.3, 19.6, 19.2, 18.8, 16.9.

### Step 2:

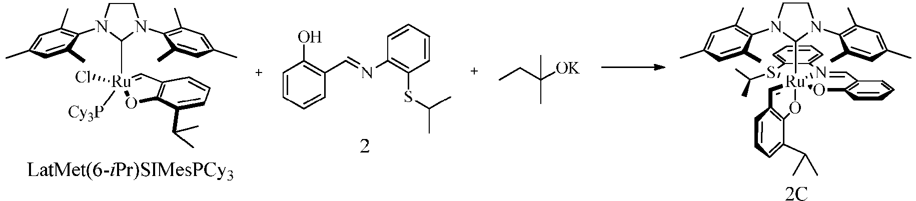

A toluene solution of potassium *tert*-pentoxide (1.7 M, 0.34 mL, 1.2 eq.) was added to a solution of imine 2 (0.157 g, 1.2 eq.) in 1,4-dioxane (5 mL) and the resulting mixture was stirred at room temperature for 30 minutes. After that, LatMet(6-*i*Pr)SIMesPCy₃ (0.42 g, 0.48 mmol, 1 eq.) was added and the reaction was stirred at 95 °C for 3 hours. The reaction mixture was filtered through a short pad of celite. The celite pad was washed with 1,4-dioxane. The solvents were evaporated to dryness. Crude product was dissolved in dichloromethane (3 mL) and heptane (15 mL). Brownish solid was filtered off and rejected. The filtrate was concentrated to dryness and crude product was crystalized from a dichloromethane/methanol mixture. Dark-brown crystals were filtered off, washed with methanol and dried, 0.19 g, 40% yield. The compound was characterized by ¹H and ¹³C NMR:
¹H NMR (CD₂Cl₂, 5.32 ppm; 600 MHz): *δ* [ppm] = 13.68 (s, 1H); 7.56 (s, 1H); 7.28-7.25 (m, 1H); 7.21-7.16 (m, 1H); 6.98-6.86 (m, 4H); 6.76-6.69 (m, 3H); 6.55-6.49 (m, 3H); 6.31-6.26 (m, 1H); 6.19-6.14 (m, 1H); 5.96-5.91 (m, 1H); 3.76-3.66 (m, 2H); 3.58-3.48 (m, 2H); 2.82 (sept, 1H, *J=* 7.2 Hz); 2.39 (s, 6H); 2.37 (s, 6H); 2.10 (s, 6H); 1.05 (d, 3H, *J=* 7.2 Hz); 0.86 (d, 3H, *J=* 7.2 Hz); 0.63 (d, 3H, *J=* 7.2 Hz); 0.55 (sept, 1H, *J=* 7.2 Hz); 0.35 (d, 3H, *J* = 7.2 Hz).
¹³C NMR (CD₂Cl₂, 54.00 ppm; 150 MHz): *δ* [ppm] = 289.1, 208.4, 179.2, 172.8, 160.9, 157.4, 151.2, 138.0, 137.7, 137.1, 136.8, 136.7, 135.3, 134.9, 133.0, 131.1, 129.2, 128.7, 128.5, 128.0, 124.4, 124.0, 123.9, 122.7, 121.2, 115.1, 111.0, 110.7, 52.3, 25.9, 24.3, 22.9, 20.8, 20.2, 18.6, 18.3, 17.8.

### Example 11

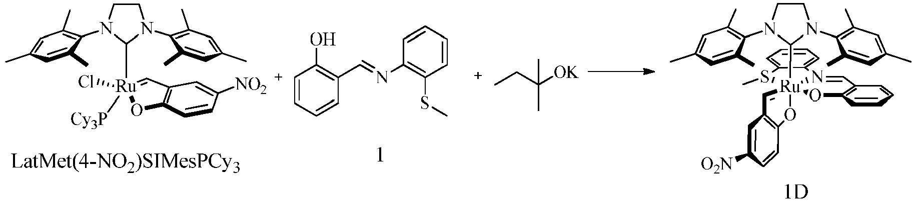

A toluene solution of potassium *tert*-pentoxide (1.7 M, 0.81 mL, 1.2 eq.) was added to a solution of imine 1 (0.33 g, 1.2 eq.) in 1,4-dioxane (11 mL) and the resulting mixture was stirred at room temperature for 30 minutes. After that, LatMet(4-NO₂)SIMesPCy₃ (1.0 g, 1.14 mmol, 1 eq.) was added and the reaction was stirred at 95 °C for 1.5 hour. The reaction mixture was cooled down to room temperature and concentrated to 40% of original volume. Crude product was filtered off, washed with toluene and methanol and recrystallized from a dichloromethane/methanol mixture, dark-green crystals, 0.72 g, 79% yield. The product was characterized by ¹H and ¹³C NMR.

Two diastereomers were observed. The minor diastereomer with the characteristic benzylidene proton signal at 14.57 ppm and the major diastereomer (95%) with the characteristic benzylidene proton signal at 14.02 ppm.

Peaks of the major diastereomer in ¹H and ¹³C NMR:
¹H NMR (CD₂Cl₂, 5.32 ppm; 600 MHz): *δ* [ppm] = 14.02 (s, 1H); 8.52 (s, 1H); 7.87-7.81 (m, 1H); 7.50-7.42 (m, 2H); 7.41-7.34 (m, 2H); 7.25-7.17 (m, 2H); 7.14-7.08 (m, 1H); 6.84 (s, 2H); 6.63 (s, 2H); 6.59-6.50 (m, 2H); 6.17 (d, 1H, *J =* 9.0Hz); 3.90-3.78 (m, 4H); 2.33 (s, 6H); 2.21 (s, 6H); 2.08 (s, 6H); 1.25 (s, 3H).
¹³C NMR (CD₂Cl₂, 54.00 ppm; 150 MHz): *δ* [ppm] = 295.5, 295.4, 211.6, 181.4, 170.6, 158.9, 151.3, 150.3, 138.9, 138.4, 137.9, 137.5, 136.9, 134.9, 133.8, 131.4, 130.6, 129.6, 129.5, 129.5, 127.6, 126.7, 123.9, 122.2, 120.2, 118.2, 117.0, 114.3, 52.5, 24.9, 21.4, 18.4, 18.3.

### Example 12

### Step 1:

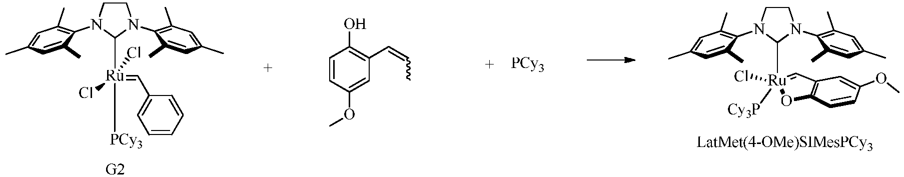

Toluene (30 mL) was added to the G2 (3 g, 3.53 mmol, 1 eq.) placed in a round bottomed flask. Next, (*E*/*Z*)-2-(prop-1-en-1-yl)-4-methoxyphenol (0.75 g, 4.59 mmol, 1.3 eq.) and tricyclohexylphosphine (1.29 g, 4.59 mmol, 1.3 eq.) were added. The reaction mixture was stirred at 80 °C for 6 hours and then cooled down to room temperature. Half of the toluene was removed on rotavapor and heptane (15 mL) was added. The sticky solid was filtered off on Celite and rejected. The filtrate was evaporated to dryness and a solid residue was further dried on high vacuum. The crude product was crystalized from a dichloromethane/methanol mixture, olive-green solid, 2.6 g, 86% yield. The compound was characterized by ¹H and ³¹P NMR:
¹H NMR (CD₂Cl₂, 5.32 ppm; 600 MHz): *δ* [ppm] = 15.59 (s, 1H), 7.06 (s, 1H), 6.69 (d, *J* = 8.4 Hz, 2H); 6.83-6.75 (m, 1H); 6.56 (d, *J* = 9.0 Hz, 1H); 6.34 (s, 1H); 5.96 (d, *J* = 3Hz, 1H); 4.02-3.93 (m, 1H); 3.83-3.69 (m, 2H); 3.67 (s, 3H); 3.65-3.58 (m, 1H); 2.61 (s, 3H); 2.53 (s, 3H); 2.51 (s, 3H); 2.34 (s, 3H); 2.28 (s, 3H); 1.72-1.38 (m, 15H); 1.29 (s, 3H); 1.14-0.97 (m, 9H), 0.97-0.66 (m, 9H).
³¹P NMR (CD₂Cl₂, 240 MHz): *δ* [ppm] = 28.45.

### Step 2:

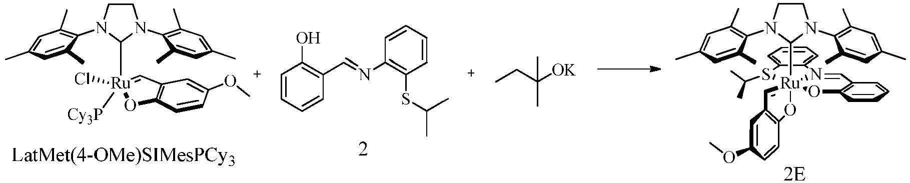

A toluene solution of potassium *tert*-pentoxide (1.7 M, 0.49 mL, 1.2 eq.) was added to a solution of imine 2 (0.23 g, 1.2 eq.) in 1,4-dioxane (7 mL) and the resulting mixture was stirred at room temperature for 30 minutes. After that, LatMet(4-OMe)SIMesPCy₃ (0.6 g, 0.7 mmol, 1 eq.) was added and the reaction was stirred at 95 °C for 5 hours. The reaction mixture was filtered through a short pad of celite. The celite pad was washed with 1,4-dioxane. The solvents were evaporated to dryness. Crude product was dissolved in dichloromethane (3 mL) and heptane (15 mL) was added. Brownish solid was filtered off and rejected. The filtrate was concentrated to *ca.* 10 mL and stored overnight in a fridge. Dark-brown crystals were filtered off and dried, 0.53 g, 93% yield. The compound was characterized by ¹H and ¹³C NMR. Two diastereomers of the product were observed. Only characteristic benzylidene protons and carbene carbons signals are provided:
¹H NMR (CD₂Cl₂, 5.32 ppm; 600 MHz): *δ* [ppm] = 13.67 (s, 0.93H), 13.60 (s, 1.06H).
¹³C NMR (CD₂Cl₂, 54.00 ppm; 150 MHz): *δ* [ppm] = 292.3, 287.4.

### Example 13

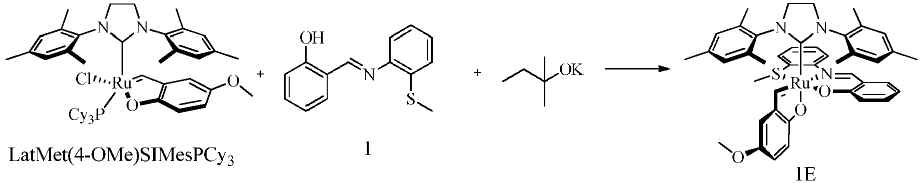

A toluene solution of potassium *tert*-pentoxide (1.7 M, 0.49 mL, 1.2 eq.) was added to a solution of imine 1 (0.20 g, 1.2 eq.) in 1,4-dioxane (7 mL) and the resulting mixture was stirred at room temperature for 30 minutes. After that, LatMet(4-OMe)SIMesPCy₃ (0.6 g, 0.7 mmol, 1 eq.) was added and the reaction was stirred at 95 °C for 3.5 hours. The reaction mixture was filtered through a short pad of celite. The celite pad was washed with 1,4-dioxane. The solvents were evaporated to dryness. Crude product was dissolved in dichloromethane (3 mL) and filtered through Celite again. Heptane (15 ml) was added and dichloromethane was removed on a rotavapor. Crude product was filtered off, washed with heptane and recrystallized from a dichloromethane/methanol mixture, dark-brown crystals, 0.44 g, 80% yield. The compound was characterized by ¹H and ¹³C NMR:
¹H NMR (CD₂Cl₂, 5.32 ppm; 600 MHz): *δ* [ppm] = 13.63 (s, 1H), 8.45 (s, 1H), 7.49 (d, 1H), 7.38-7.31 (m, 2H), 7.19-7.10 (m, 2H), 7.05 (ddd, 1H), 6.79 (bs, 2H), 6.73 (dd, 1H), 6.65 (bs, 2H), 6.50 (d, 1H), 6.44 (ddd, 1H), 6.20 (d, 1H), 6.00 (d, 1H), 3.87-3.75 (m, 4H), 3.66 (s, 3H), 2.30 (s, 6H), 2.21 (s, 6H), 2.08 (s, 6H), 1.24 (s, 3H).
¹³C NMR (CD₂Cl₂, 54.00 ppm; 150 MHz): *δ* [ppm] = 293.4, 215.4, 174.2, 170.4, 157.9, 151.5, 150.9, 148.5, 139.6, 138.0, 137.7, 137.5, 136.8, 133.1, 132.9, 130.6, 129.4, 129.3, 129.1, 126.0, 124.2, 122.8, 122.3, 118.4, 118.1, 113.3, 106.3, 56.2, 52.6, 22.9, 21.4, 18.6, 18.5.

## Claims

1. A compound of the formula II, including all possible enantiomers, diastereomers, geometric or any other stereoisomers thereof: 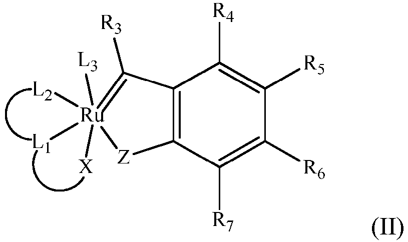 wherein:
Z is oxygen;
R₃ is hydrogen;
R₄, R₅, R₆ and R₇ are the same or different and each independently selected from the group consisting of hydrogen, methyl, ethyl and -NO₂; 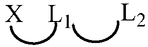 is a group of the formula (III): 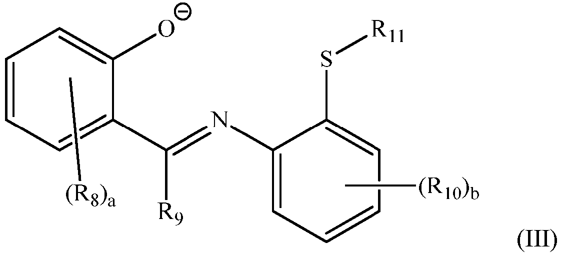 wherein:
a and b are integers from 0 to 5;
each R₈, R₉, R₁₀ and R₁₁ may be the same or different and independently of the other selected from the group consisting of hydrogen, halogen, (C₁-C₁₆)alkyl, (C₁-C₁₆)alkoxy, (C₁-C₁₆)perhaloalkyl, (C₃-C₇)cycloalkyl, (C₂-C₁₆)alkenyl, (C₆-C₁₄)aryl, (C₆-C₁₄)perhaloaryl, (C₃-C₁₂)heterocyclyl, (C₄-C₂₀)alkylaryl, -OR₁₈, -NO₂, -COOH,
-COOR₁₈, -CONR₁₈R₁₉, -SO₂NR₁₈R₁₉, -SO₂R₁₅, -CHO, -COR₁₈, wherein R₁₈ and R₁₉ are the same or different and each independently selected from the group consisting of (C₁-C₆)alkyl, (C₁-C₆)perhaloalkyl, (C₆-C₁₄)aryl, (C₆-C₁₄)perhaloaryl;
L₃ is an N-heterocyclic carbene ligand of the formula (IVA) or (IVB): 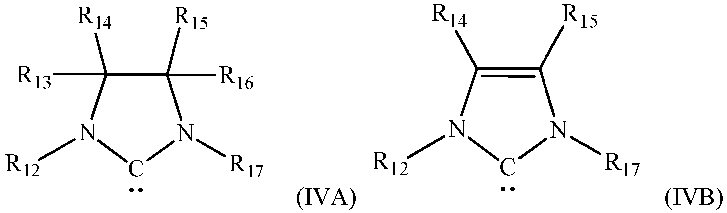 wherein:
R₁₂ and R₁₇ are the same or different and each independently selected from the group consisting of (C₁-C₁₂)alkyl, (C₃-C₁₂)cycloalkyl, (C₂-C₁₂)alkenyl and (C₆-C₁₄)aryl unsubstituted or substituted by (C₁-C₄)alkyl, (C₁-C₁₆)perhaloalkyl, (C₃-C₇)cycloalkyl or (C₄-C₂₀)alkylaryl;
R₁₃, R₁₄, R₁₅ and R₁₆ are the same or different and each independently selected from the group consisting of hydrogen, (C₁-C₁₂)alkyl, (C₃-C₁₂)cycloalkyl, (C₂-C₁₂)alkenyl, (C₆-C₁₄)aryl, optionally substituted with at least one of (C₁-C₆)alkyl, (C₁-C₆)perhaloalkyl, (C₁-C₆)alkoxy or halogen; or
R₁₃, R₁₄, R₁₅, R₁₆ may optionally join together with the carbon atoms to which they are attached to form a fused (C₄-C₈)carbocyclic ring unsubstituted or substituted by (C₁-C₄)alkyl, (C₁-C₁₆)perhaloalkyl, (C₃-C₇)cycloalkyl or (C₄-C₂₀)alkylaryl, or a fused aromatic ring unsubstituted or substituted by (C₁-C₄)alkyl, (C₁-C₁₆)perhaloalkyl, (C₃-C₇)cycloalkyl or (C₄-C₂₀)alkylaryl.

2. The compound according to claim 1, wherein: 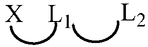 selected from the group consisting of:
a group of the formula (IIIA): 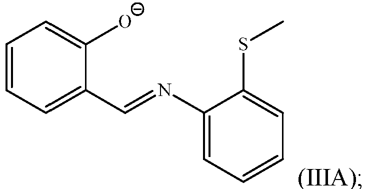
a group of the formula (IIIB): 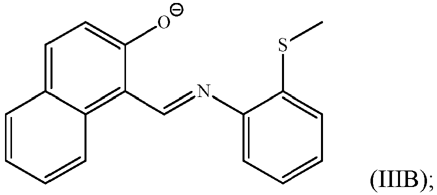
a group of the formula (IIIC): 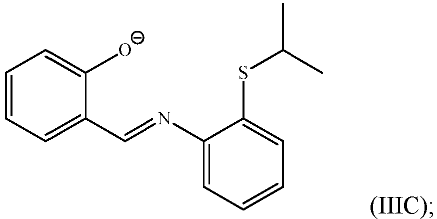
a group of the formula (IIID): 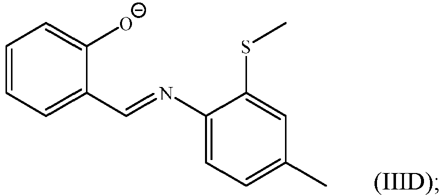
a group of the formula (IIIE): 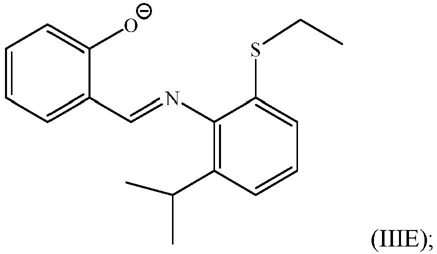
a group of the formula (IIIF): 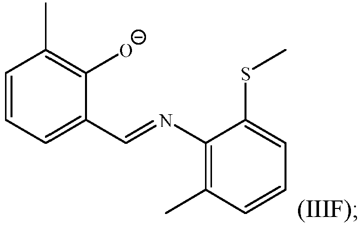 and
a group of the formula (IIIG):

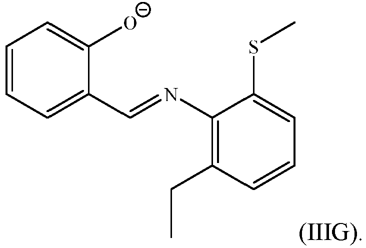

3. The compound according to claim 1, wherein:
L₃ is selected from the group consisting of:

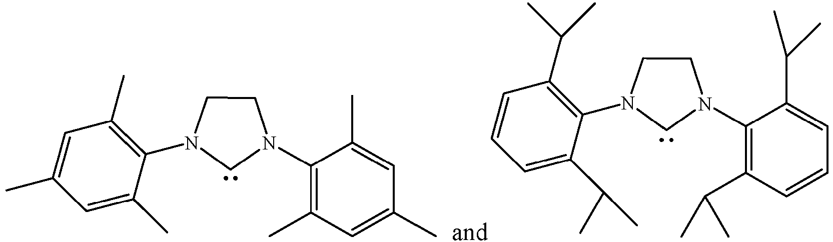

4. The compound according to claim 1, which is selected from the group consisting of:

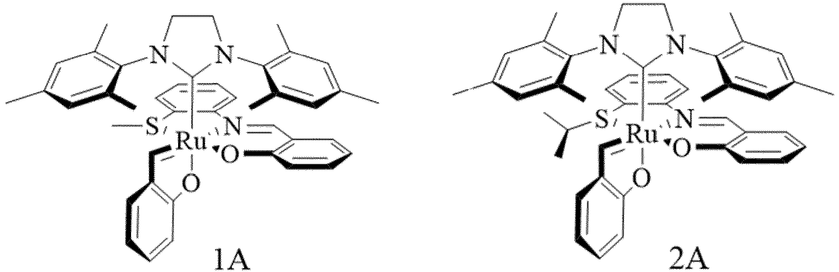

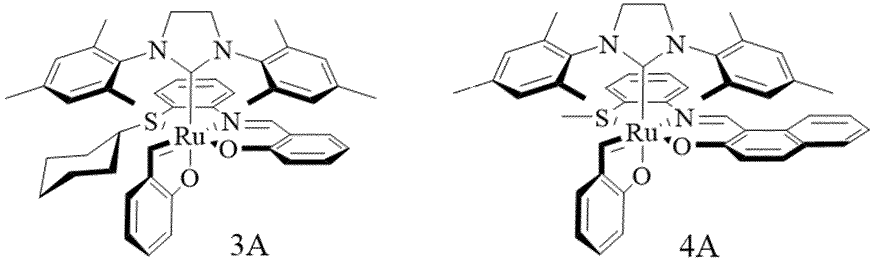

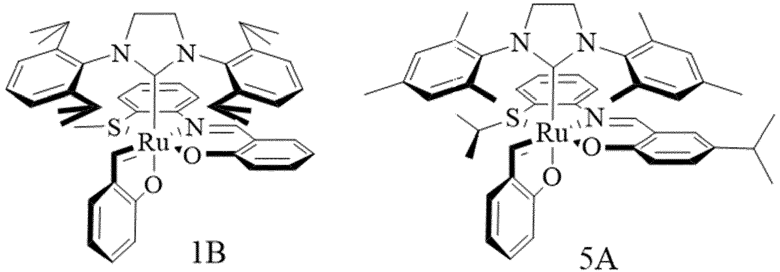

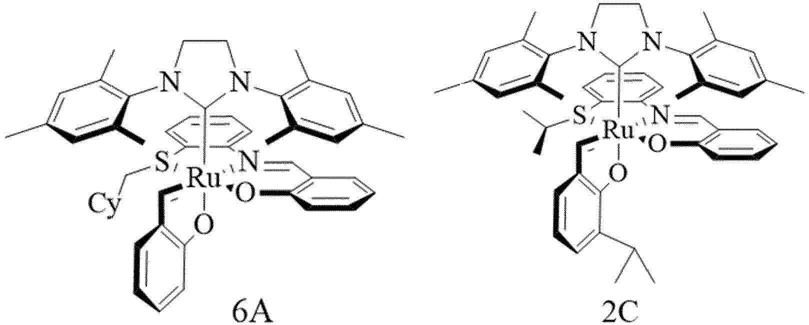

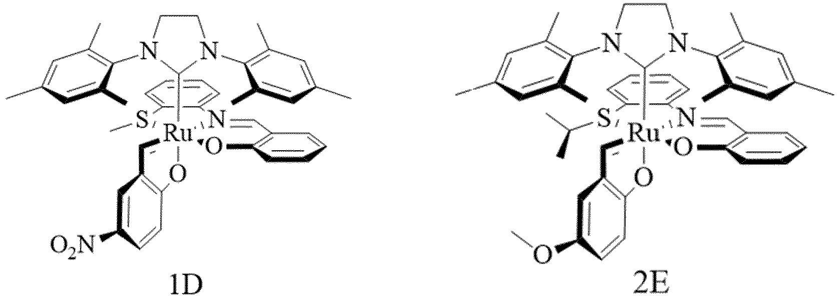

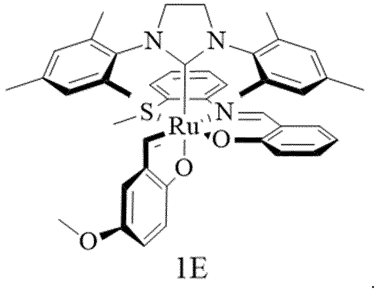

5. A process for carrying out a metathesis reaction of olefins, comprising contacting at least one olefin with the compound of claim 1 as a (pre)catalyst.

6. The process according to claim 5, wherein the metathesis reaction is carried out in an organic solvent.

7. The process according to claim 5, wherein the metathesis reaction is carried out without any solvent.

8. The process according to any of the claims 5-7, wherein the metathesis reaction is carried out in the presence of a chemical activator.

9. The process according to claim 8, wherein the activator is hydrogen chloride, chlorotrimethylsilane or p-toluenesulfonic acid.

10. The process according to any of the claims 5-9, wherein the metathesis reaction is a ring-opening metathetic polymerization of dicyclopentadiene.

11. The process according to claim 10, wherein the (pre)catalyst of the general formula II is added in the solid form to dicyclopentadiene.

12. The process according to claim 10 or 11, wherein the polymerization reaction is initiated by heating the mixture of dicyclopentadiene and the (pre)catalyst of the general formula II to a temperature of 30° C or higher.

13. The process according to any of the claims 5-12, wherein the metathesis reaction is carried out in the presence of an additive promoting formation of cross bonds.

## Patentansprüche

1. Eine Verbindung der Formel II, einschließlich aller möglichen Enantiomere, Diastereomere, geometrischen Isomere oder sonstigen Stereoisomere davon: 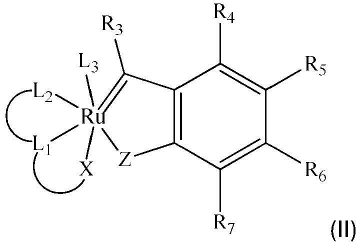 wobei:
Z Sauerstoff ist;
R₃ Wasserstoff ist;
R₄, R₅, R₆ und R₇ gleich oder verschieden sind und jeweils unabhängig ausgewählt sind aus der Gruppe bestehend aus Wasserstoff, Methyl, Ethyl und NO₂; 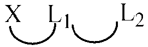 eine Gruppe der Formel (III) ist: 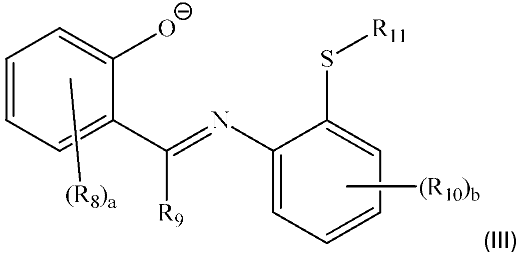
wobei:
a und b ganze Zahlen von 0 bis 5 sind;
jedes R₈, R₉, R₁₀ und R₁₁ gleich oder verschieden sein kann und unabhängig von den anderen ausgewählt ist aus der Gruppe bestehend aus Wasserstoff, Halogen, (C₁-C₁₆)Alkyl, (C₁-C₁₆)Alkoxy, (C₁-C₁₆)Perhaloalkyl, (C₃-C₇)Cycloalkyl, (C₂-C₁₆)Alkenyl, (C₆-C₁₄)Aryl, (C₆-C₁₄)Perhatoaryl, (C₃-C₁₂)Heterocyctyl, (C₄-C₂₀)Alkylaryl, OR₁₈, NO₂, COOH, COOR₁₈, CONR₁₈R₁₉, SO₂NR₄₈R₁₂, -SO₂R₁₈, -CHO, -COR₁₈, wobei R₁₈ und R₁₉ gleich oder verschieden sind und jeweils unabhängig ausgewählt sind aus der Gruppe bestehend aus (C₁-C₆)Alkyl, (C₁-C₆)Perhatoalkyl, (C₆-C₁₄)Aryl, (C₆-C₁₄)Perhatoaryl;
L₃ ein N-heterocyclischer Carbenligand der Formel (IVA) oder (IVB) ist: 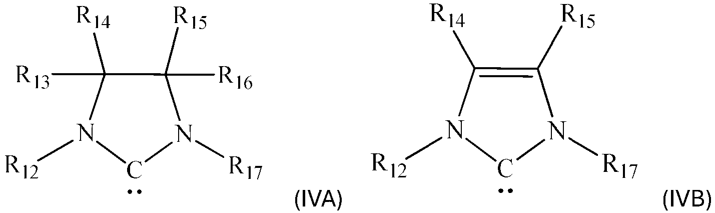 wobei:
R₁₂ und R₁₇ gleich oder verschieden sind und jeweils unabhängig ausgewählt sind aus der Gruppe bestehend aus unsubstituiertem oder substituiertem (C₁-C₁₂)Alkyl, (C₃-C₁₂)Cycloalkyl, (C₂-C₁₂)Alkenyl und (C₆-C₁₄)Aryl, substituiert durch (C₁-C₄)Alkyl, (C₁-C₁₆)Perhaloalkyl, (C₃-C₇)Cycloalkyl oder (C₄-C₂₀)Alkylaryl;
R₁₃, R₁₄, R₁₅ und R₁₆ gleich oder verschieden sind und jeweils unabhängig ausgewählt sind aus der Gruppe bestehend aus Wasserstoff, (C₁-C₁₂)Alkyl, (C₃-C₁₂)Cycloalkyl, (C₂-C₁₂)Alkenyl, (C₆-C₁₄)Aryl, optional substituiert mit mindestens einem aus (C₁-C₆)Alkyl, (C₁-C₆)Perhaloalkyl, (C₁-C₆)Alkoxy oder Halogen; oder
R₁₃, R₁₄, R₁₅ und R₁₆ optional gemeinsam mit den Kohlenstoffatomen, an die sie gebunden sind, einen kondensierten unsubstituierten oder substituierten (C₄-C₈)carbocyclischen Ring bilden können, substituiert durch (C₁-C₄)Alkyl, (C₁-C₁₆)Perhaloalkyl, (C₃-C₇)Cycloalkyloder (C₄-C₂₀)Alkylaryl, oder einen kondensierten aromatischen Ring, unsubstituiert oder substituiert durch (C₁-C₄)Alkyl, (C₁-C₁₆)Perhaloalkyl, (C₃-C₇)Cycloalkyloder (C₄-C₂₀)Alkylaryl.

2. Die Verbindung nach Anspruch 1, wobei: 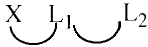 ausgewählt aus der Gruppe bestehend aus:
eine Gruppe der Formel (IIIA): 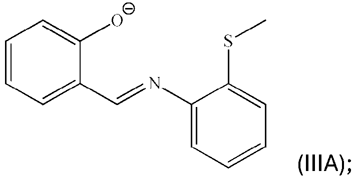
eine Gruppe der Formel (IIIB): 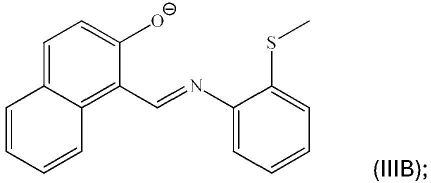
eine Gruppe der Formel (IIIC): 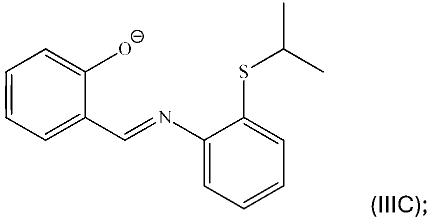
eine Gruppe der Formel (IIID): 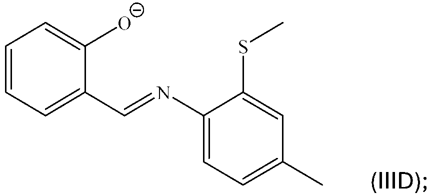
eine Gruppe der Formel (IIIE): 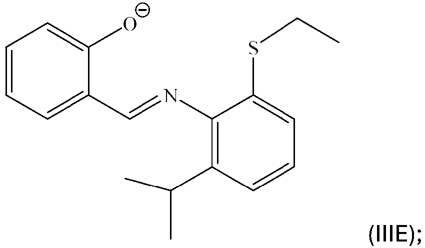
eine Gruppe der Formel (IIIF): 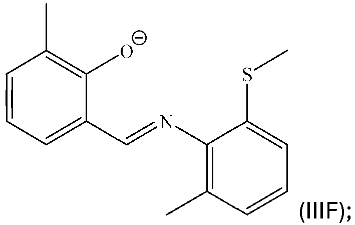 and
eine Gruppe der Formel (IIIG):

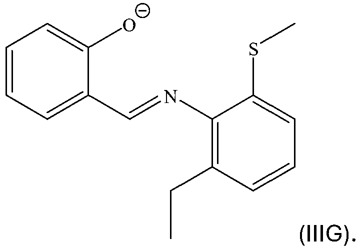

3. Die Verbindung nach Anspruch 1, wobei:
L₃ ausgewählt ist aus der Gruppe bestehend aus:

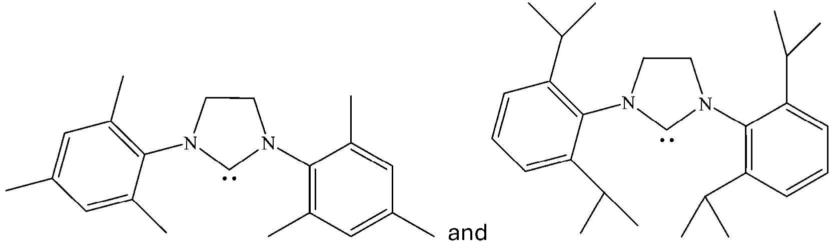

4. Die Verbindung nach Anspruch 1, ausgewählt aus der Gruppe bestehend aus:

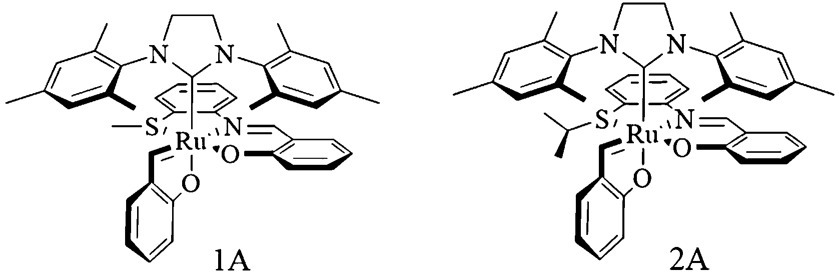

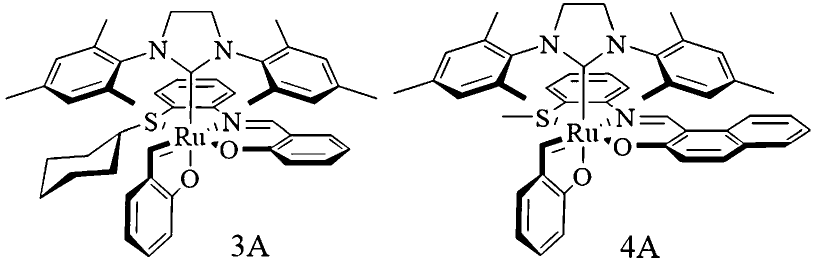

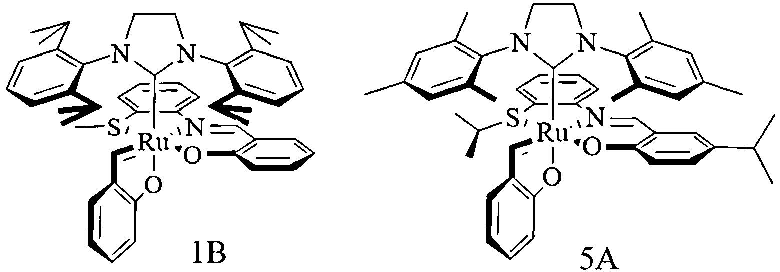

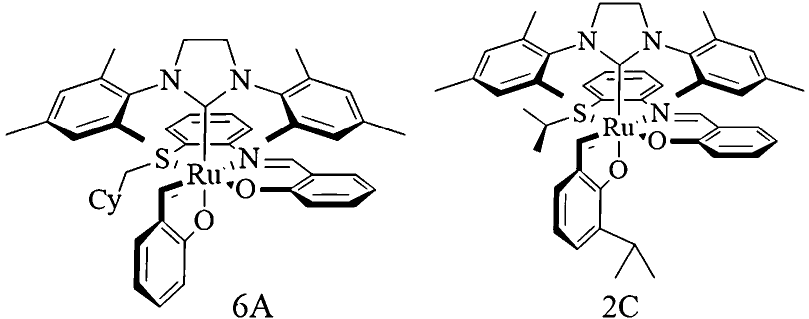

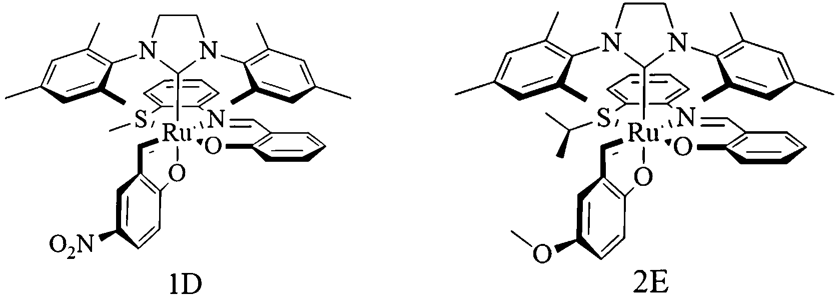

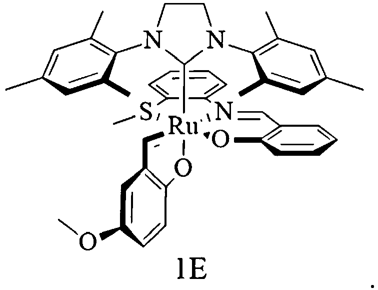

5. Verfahren zur Durchführung einer Metathesereaktion von Olefinen, umfassend das In-Kontakt-Bringen mindestens eines Olefins mit der Verbindung nach Anspruch 1 als (Prä-)Katalysator.

6. Verfahren nach Anspruch 5, wobei die Metathesereaktion in einem organischen Lösungsmittel durchgeführt wird.

7. Verfahren nach Anspruch 5, wobei die Metathesereaktion ohne Lösungsmittel durchgeführt wird.

8. Verfahren nach einem der Ansprüche 5 bis 7, wobei die Metathesereaktion in Gegenwart eines chemischen Aktivators durchgeführt wird.

9. Verfahren nach Anspruch 8, wobei der Aktivator Chlorwasserstoff, Chlortrimethylsilan oder p-Toluolsulfonsäure ist.

10. Verfahren nach einem der Ansprüche 5 bis 9, wobei es sich bei der Metathesereaktion um eine ringöffnende metathetische Polymerisation von Dicyclopentadien handelt.

11. Verfahren nach Anspruch 10, wobei der (Prä-)Katalysator der allgemeinen Formel 1 in fester Form zu Dicyclopentadien gegeben wird.

12. Verfahren nach Anspruch 10 oder 11, wobei die Polymerisationsreaktion durch Erhitzen des Gemisches aus Dicyclopentadien und dem (Prä-)Katalysator der allgemeinen Formel 1 auf eine Temperatur von 30 °C oder höher initiiert wird.

13. Verfahren nach einem der Ansprüche 5 bis 12, wobei die Metathesereaktion in Gegenwart eines Additivs durchgeführt wird, das die Bildung von Querbindungen fördert.

## Revendications

1. Un composé de formule II, y compris tous les énantiomères, diastéréoisomères, isomères géométriques ou tout autre stéréoisomère possibles de celui-ci : 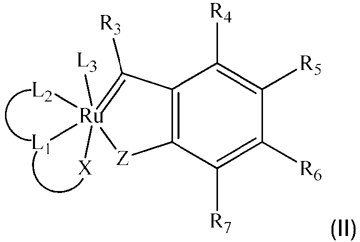 où:
Z est l'oxygène;
R₃ est l'hydrogène;
R₄, R₅, R₆ et R₇ sont identiques ou différents et chacun est indépendamment sélectionné dans le groupe constitué de l'hydrogène, du méthyle, de l'éthyle et de -NO₂; 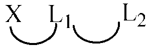 est un groupe de formule (III): 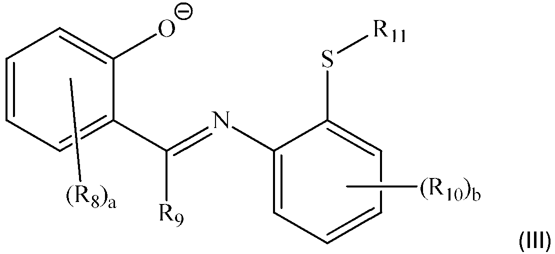 où:
a et b sont des entiers de 0 à 5 ;
chaque R₈, R₉, R₁₀ et R₁₁ peut être identique ou différent et est sélectionné indépendamment des autres dans le groupe constitué de l'hydrogène, d'un halogène, d'un alkyle en (C₁-C₁₆), d'un alcoxy en (C₁-C₁₆), d'un perhaloalkyle en (C₁-C₁₆), d'un cycloalkyle en (C₃-C₇), d'un alcényle en (C₂-C₁₆), d'un aryle en (C₆-C₁₄), d'un perhaloaryle en (C₆-C₁₄), d'un hétérocyclyle en (C₃-C₁₂), d'un alkylaryle en (C₄-C₂₀), -OR₁₈, -NO₂, -COOH, -COOR₁₈, -CONR₁₈R₁₉, -SO₂NR₁₈R₁₉, -SO₂R₁₈, -CHO, -COR₁₈, où R₁₈ et R₁₉ sont identiques ou différents et chacun est indépendamment sélectionné dans le groupe constitué de (C₁-C₆)alkyle, (C₁-C₆)perhaloalkyle, (C₆-C₁₄)aryle, (C₆-C₁₄)perhaloaryle;
L₃ est un ligand carbène N-hétérocyclique de formule (IVA) ou (IVB): 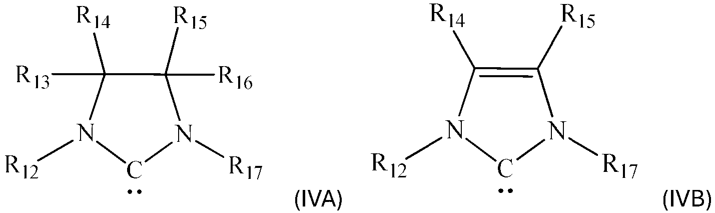 où:
R₁₂ et R₁₇ sont identiques ou différents et chacun est indépendamment sélectionné dans le groupe constitué de (C₁-C₆)alkyle, (C₃-C₁₂)cycloalkyle, (C₂-C₁₂)alcényle et (C₆-C₁₄)aryle non substitué ou substitué par (C₁-C₄)alkyle, (C₁-C₁₆)perhaloalkyle, (C₃-C₇)cycloalkyle ou (C₄-C₂₀)alkylaryle
R₁₃, R₁₄, R₁₅ et R₁₆ sont identiques ou différents et chacun est indépendamment sélectionné dans le groupe constitué de hydrogène, (C₁-C₁₂)alkyle, (C₃-C₁₂)cycloalkyle, (C₂-C₁₂)alcényle, (C₆-C₁₄)aryle, éventuellement substitué par au moins un (C₁-C₆)alkyle, (C₁-C₆)perhaloalkyle, (C₁-C₆)alcoxy ou halogène ; ou
R₁₃, R₁₄, R₁₅, R₁₆ peuvent éventuellement être réunis avec les atomes de carbone auxquels ils sont liés pour former un cycle carbocyclique condensé en (C₄-C₈), non substitué ou substitué par (C₁-C₄)alkyle, (C₁-C₁₆)perhaloalkyle, (C₃-C₇)cycloalkyle ou (C₄-C₂₀)alkylaryle, ou un cycle aromatique condensé, non substitué ou substitué par (C₁-C₄)alkyle, (C₁-C₁₆)perhaloalkyle, (C₃-C₇)cycloalkyle ou (C₄-C₂₀)alkylaryle.

2. Le composé selon la revendication 1, **caractérisé en ce que**: 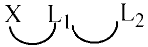 sélectionné dans le groupe constitué de:
un groupe de formule (IIIA): 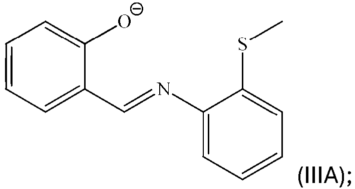
un groupe de formule (IIIB): 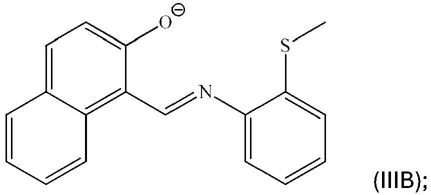
un groupe de formule (IIIC): 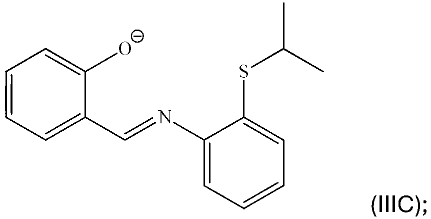
un groupe de formule (IIID): 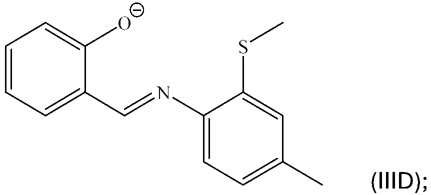
un groupe de formule (IIIE): 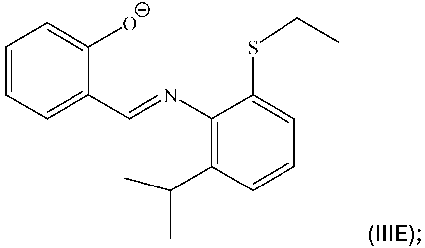
un groupe de formule (IIIF): 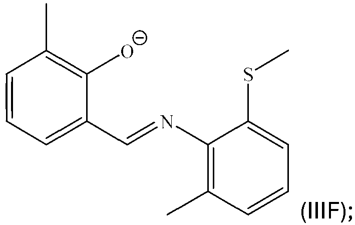 and
un groupe de formule (IIIG):

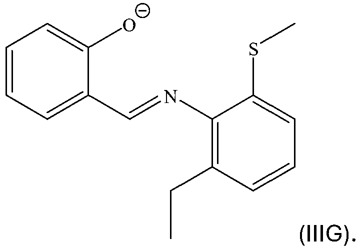

3. Le composé selon la revendication 1, **caractérisé en ce que**:
L₃ est sélectionné dans le groupe constitué de:

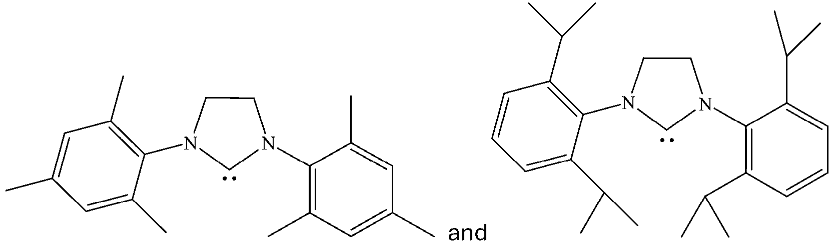

4. Le composé selon la revendication 1, qui est sélectionné dans le groupe constitué de:

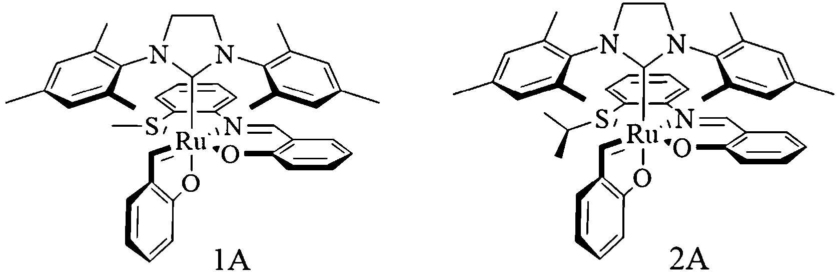

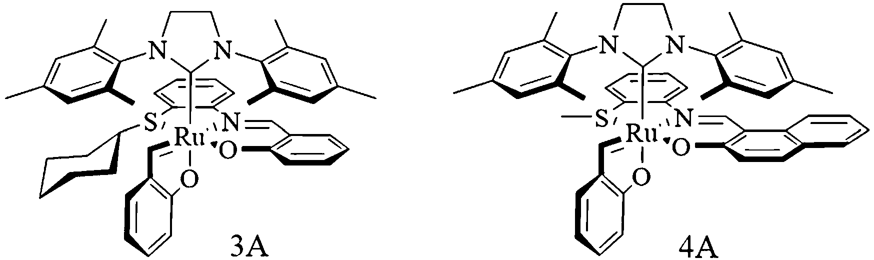

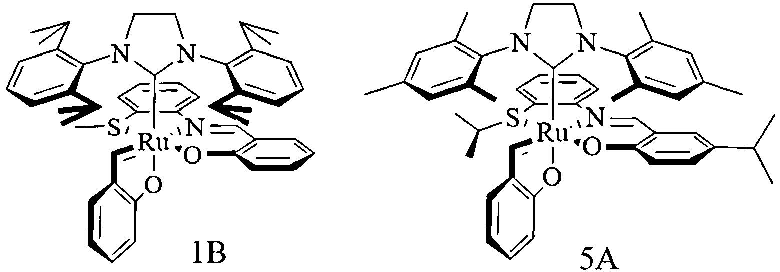

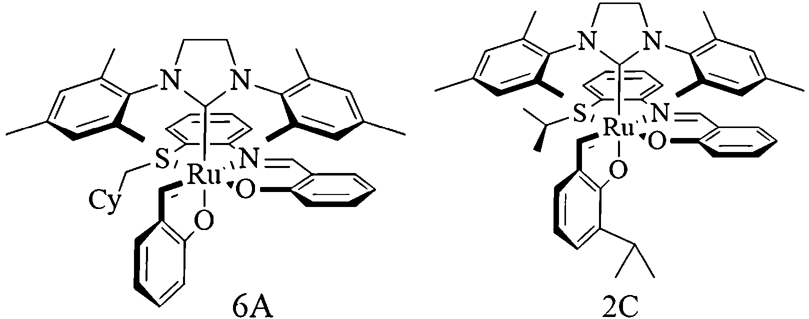

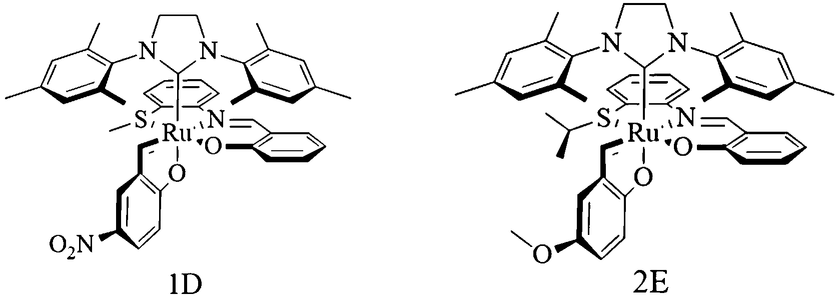

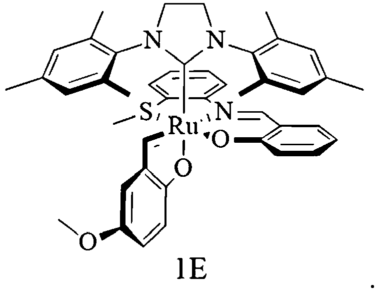

5. Procédé de réalisation d'une réaction de métathèse d'oléfines, comprenant la mise en contact d'au moins une oléfine avec le composé selon la revendication 1 en tant que (pré)catalyseur.

6. Procédé selon la revendication 5, **caractérisé en ce que** la réaction de métathèse est réalisée dans un solvant organique.

7. Procédé selon la revendication 5, **caractérisé en ce que** la réaction de métathèse est réalisée sans aucun solvant.

8. Procédé selon l'une quelconque des revendications 5 à 7, **caractérisé en ce que** la réaction de métathèse est réalisée en présence d'un activateur chimique.

9. Procédé selon la revendication 8, **caractérisé en ce que** l'activateur est le chlorure d'hydrogène, le chlorotriméthylsilane ou l'acide p-toluènesulfonique.

10. Procédé selon l'une quelconque des revendications 5 à 9, **caractérisé en ce que** la réaction de métathèse est une polymérisation par métathèse avec ouverture de cycle du dicyclopentadiène.

11. Procédé selon la revendication 10, **caractérisé en ce que** le (pré)catalyseur de formule générale 1 est ajouté sous forme solide au dicyclopentadiène.

12. Procédé selon la revendication 10 ou 11, **caractérisé en ce que** la réaction de polymérisation est initiée par chauffage du mélange de dicyclopentadiène et du (pré)catalyseur de formule générale 1 à une température de 30 °C ou plus.

13. Procédé selon l'une quelconque des revendications 5 à 12, **caractérisé en ce que** la réaction de métathèse est réalisée en présence d'un additif favorisant la formation de liaisons croisées
